# EUROPÄISCHE PATENTANMELDUNG

(11) **EP 2 105 441 A1**
(43) Veröffentlichungstag der Anmeldung: **30.09.2009**
(21) Anmeldenummer: 09160132.8
(22) Anmeldetag: 15.10.2004
(51) Int. Cl.: C07D 317/60, C07D 401/14, C07D 403/06, C07D 409/14, C07D 413/06, C07D 413/14, A61K 31/36, A61K 31/4166, A61K 31/4439, A61K 31/445, A61K 31/496, A61K 31/5377, A61P 25/00

(54) **Duale Alanyl-Aminopeptidase- und Dipeptidylpeptidase IV-Inhibitoren zur Behandlung immunologischer, entzündlicher, neuronaler und anderer Erkrankungen**

(30) Priorität: 15.10.2003 DE 10348044
(62) Teilanmeldung aus: 04790486.7
(71) Anmelder: IMTM GmbH, 39120 Magdeburg (DE); KeyNeurotek Pharmaceuticals AG, 39120 Magdeburg (DE)
(72) Erfinder: Ansorge, Siegfried, 39291 Hohenwarthe (DE); Bank, Ute, 39418 Stassfurt (DE); Nordhoff, Karsten, 39118 Magdeburg (DE); Täger, Michael, 39326 Heinrichsberg (DE); Striggow, Frank, 39175 Gerwisch (DE)
(74) Vertreter: Koepe & Partner

(57) **Zusammenfassung**

Die vorliegende Erfindung betrifft Substanzen, die sowohl Ala-p-Nitroanilid spaltende Peptidasen als auch Gly-Pro-p-Nitroanilid spaltende Peptidasen spezifisch inhibieren, für die Verwendung in der Medizin. Weiter betrifft die Erfindung die Verwendung mindestens einer derartigen Substanz oder mindestens einer mindestens eine derartige Substanz enthaltenden pharmazeutischen oder kosmetischen Zusammensetzung zur Prophylaxe und Therapie von Erkrankungen, insbesondere zur Prophylaxe und Therapie von Erkrankungen mit überschießender Immunantwort (Autoimmunerkrankungen, Allergien und Transplantatrejektionen), von anderen chronisch-entzündlichen Erkrankungen, neuronalen Erkrankungen und zerebralen Schädigungen, Hauterkrankungen (u. a. Akne und Schuppenflechte), Tumorerkrankungen und speziellen Virusinfektionen (u. a. SARS).

## Beschreibung

Die Erfindung beschreibt neuartige Stoffe, die in der Lage sind, die Enzyme Dipeptidylpeptidase IV sowie Peptidasen mit analoger enzymatischer Wirkung und die Alanyl-Aminopeptidasen sowie Peptidasen mit analoger enzymatischer Wirkung gemeinsam zu inhibieren. Die Erfindung beschreibt darüber hinaus die Verwendung dieser dualen Inhibitoren zur Prophylaxe und Therapie von Erkrankungen mit überschießender Immunantwort und entzündlicher Genese, neuronalen Erkrankungen und zerebralen Schädigungen sowie Tumorerkrankungen.

Die Dipeptidylpeptidase IV (DPIV, CD26, EC 3.4.14.5) ist eine ubiquitär auftretende Serin-Protease, die die Hydrolyse von Peptiden spezifisch hinter Prolin oder Alanin in der zweiten Position des N-Terminus katalysiert. Zur Gen-Familie der DPIV mit enzymatischer Aktivität gehören u. a. DP 8, DP 9 und FAP/Seprase (T. Chen et al.: Adv.Exp. Med. Biol. 524, 79, 2003). Eine ähnliche Substratspezifität wie DPIV weist Attractin (mahagony protein) auf (J. S. Duke-Cohan et al.: J. Immunol. 156, 1714, 1996). Das Enzym wird ebenfalls durch DPIV-Inhibitoren gehemmt.

Zur Gruppe der ebenfalls ubiquitär vorkommenden Alanyl-Aminopeptidasen gehören die überwiegend als TypII-Membranprotein auftretende Aminopeptidase N (APN, CD13, EC 3.4.11.2) sowie die zytosolische, lösliche Alanyl-Aminopeptidase (EC 3.4.11.14, Puromycinsensitive Aminopeptidase, Aminopeptidase PS, Enkephalin-abbauende Aminopeptidase). Beide Peptidasen wirken Metall-abhängig und katalysieren die Hydrolyse der Peptidbindungen hinter N-terminalen Aminosäuren von Oligopeptiden, im Falle der APN mit einer Bevorzugung von Alanin am N-Terminus (A. J. Barrett et al.: Handbook of Proteolytic Enzymes, Academic Press 1998). Alle Hemmstoffe der Aminopeptidase N hemmen auch die zytosolische Alanyl-Aminopeptidase, dagegen existieren spezifische Inhibitoren der zytosolischen Aminopeptidase (M. Komodo et al.: Bioorg. and Med. Chem. 9, 121, 2001).

Für beide Enzymgruppen wurden wichtige biologische Funktionen in unterschiedlichen Zellsystemen nachgewiesen. Dies gilt u. a. für das Immunsystem (U. Lendeckel et al.: Intern. J. Mol. Med. 4, 17, 1999; T. Kähne et al.: Intern. J. Mol. Med. 4, 3, 1999; I. De Meester et al: Advanc. Exp. Med. Biol. 524, 3, 2002; Internationale Patentanmeldung WO 01/89569 C1; Internationale Patentanmeldung WO 02/053170 A3 ; Internationale Patentanmeldung PCT/EP 03/07199), das neuronale System (Internationale Patentanmeldung WO 02/053169 A2 und Deutsche Patentanmeldung 103 37 074.9), die Fibroblasten (Deutsche Patentanmeldung 103 30 842.3), die Keratinozyten (Internationale Patentanmeldung WO 02/053170 A3), die Talgdrüsenzellen/Sebozyten (Internationale Patentanmeldung PCT/EP 03/02356), Tumore sowie für Infektionen durch Viren, z.B. Coronaviren ( D. P. Kontoyiannis et al.: Lancet 361, 1558,2003).

Die Fähigkeit der DPIV, die inkretorischen Hormone GIP und GLP spezifisch zu Inactiveieren, hat zur Entwicklung eines neuen therapeutischen Konzepts zur Behandlung von Glukose-Stoffwechselstörungen geführt (D. M. Evans: Drugs 5, 577, 2002).

Für beide Enzymgruppen sind unterschiedliche Inhibitoren bekannt (Reviews in D. M. Evans: Drugs 5, 577, 2002 und M.-C. Fournie-Zaluski und B. P. Roques: in J. Langner and S. Ansorge, Ectopeptidases, Kluwer Academic/Plenum Publishers, P. 51, 2002).

Die isolierte Hemmung der Alanyl-Aminopeptidasen und der Dipeptidylpeptidase IV-Gruppe, insbesondere aber die kombinierte Hemmung beider Enzymgruppen führt an Immunzellen zur starken Hemmung der DNA-Synthese und damit der Zellvermehrung sowie zur Veränderung der Zytokinproduktion, insbesondere zur Induktion des immunregulatorisch wirkenden TGF-β1 (Internationale Patentanmeldung WO 01/89569 C1, Internationale Patentanmeldung WO 02/053170 A3). An regulatorischen T-Zellen bewirken Alanyl-Aminopeptidase-Inhibitoren eine starke Induktion von TGF-β1 (Internationale Patentanmeldung PCT/EP 03/07199). Im neuronalen System wurde durch Hemmung der beiden Enzymsysteme eine Verminderung bzw. Verzögerung akuter und chronischer zerebraler Schädigungsprozesse nachgewiesen (Internationale Patentanmeldung WO 02/053169 A3 und Deutsche Patentanmeldung 103 37 074.9). Auch an Fibroblasten (Deutsche Patentanmeldung 103 30 842.3), Keratinozyten (Internationale Patentanmeldung WO 02/0531 70 A3) und Sebozyten (Internationale Patentanmeldung PCT/EP 03/02356) wurde gezeigt, dass die kombinierte Hemmung der Alanyl-Aminopeptidasen und DPIV eine Hemmung des Wachstums und eine Veränderung der Zytokinproduktion bewirkt.

Damit ergibt sich der überraschende Sachverhalt, dass die Alanyl-Aminopeptidasen und die Dipeptidylpeptidase IV sowie analog wirkende Enzyme fundamentale zentrale biologische Funktionen in unterschiedlichen Organen und Zellsystemen erfüllen und eine kombinierte Hemmung beider Enzymgruppen ein neues wirkungsvolles therapeutisches Prinzip für die Behandlung unterschiedlichster, zumeist chronischer Erkrankungen repräsentiert.

An akzeptierten Tiermodellen konnten die Anmelder inzwischen zeigen, dass insbesondere die kombinierte Gabe von Inhibitoren der beiden Peptidase-Gruppen in der Tat auch in vivo eine Hemmung des Wachstums verschiedener Zellsysteme und eine Unterdrückung einer überschießenden Immunantwort, chronisch-entzündlicher Vorgänge sowie zerebraler Schädigungen bewirkt (Internationale Patentanmeldung WO 01/89569 C1). Die Gabe einzelner bekannter Inhibitoren zeigte eine schwächere oder keine Wirkung.

Die bisherigen Ergebnisse wurden überwiegend mit Hilfe bekannter, in der Literatur beschriebener und z.T. kommerziell zugänglicher Inhibitoren der Alanyl-Aminopeptidasen und der Dipeptidylpeptidase IV allein, besonders aber in Kombination der Inhibitoren beider Enzymgruppen, erhalten.

Im Rahmen eines high-throughput-screenings von Substanzbanken wurden nun überraschend neuartige, überwiegend nicht-peptidische, niedermolekulare, duale Inhibitoren für die Gruppe der Alanyl-Aminopeptidasen und der Dipeptidylpeptidase IV gefunden.

Die Erfindung betrifft neue Substanzen, die sowohl Ala-p-Nitroanilid als auch Gly-Pro-p-Nitroanilid spaltende Peptidasen spezifisch inhibieren und damit die Fähigkeit der kombinierten Hemmung beider Peptidase-Gruppen in jeweils einer Substanz vereinen.

Die Erfindung betrifft darüber hinaus neue Stoffe, die als solche oder als Ausgangsstoffe für weitere Substanzen zur Therapie von Erkrankungen mit überschießender Immunantwort (Autoimmunerkrankungen, Allergien und Transplantatrejektionen, Sepsis) anderen chronisch-entzündlichen Erkrankungen, neuronalen Erkrankungen und zerebralen Schädigungen, Hauterkrankungen (u. a. Akne und Schuppenflechte), Tumorerkrankungen und speziellen Virusinfektionen (u. a. SARS) genutzt werden können.

Insbesondere betrifft die vorliegende Erfindung Verbindungen der allgemeinen Formeln C1 bis C16 nach den Patentansprüchen 1, 3, 5, 7, 9, 11, 13, 15, 17, 19, 21, 23, 25, 27, 29, 30a und 30b sowie Tautomere und Stereoisomere der genannten Verbindungen der allgemeinen Formeln C1 bis C16 und pharmazeutisch annehmbare Salze, Salzderivate, Tautomere und Stereoisomere davon, für die Verwendung in der Medizin.

In besonderen Ausführungsformen betrifft die Erfindung spezielle, unter die obigen allgemeinen Formeln C1 bis C16 fallende, bevorzugte Verbindungen der besonderen Formeln C1.001 bis C16.013, die beispielhaft, jedoch nicht beschränkend in den Patentansprüchen 2, 4, 6, 8, 10, 12, 14, 16, 18, 20, 22, 24, 26, 28, 30 und 30c in Form von Tabellen aufgelistet sind, sowie Tautomere und Stereoisomere der genannten Verbindungen der allgemeinen Formeln C1.001 bis C16.013 und pharmazeutisch annehmbare Salze, Salzderivate, Tautomere und Stereoisomere davon, für die Verwendung in der Medizin.

Die Erfindung betrifft weiter pharmazeutische Zusammensetzungen, die mindestens eine Verbindung einer der allgemeinen Formeln C1 bis C16 umfassen, gegebenenfalls in Kombination mit an sich üblichen Trägern oder Adjuvantien.

Die Erfindung betrifft weiter kosmetische Zusammensetzungen, die mindestens eine Verbindung einer der allgemeinen Formeln C1 bis C16 umfassen, gegebenenfalls in Kombination mit an sich üblichen Trägern oder Adjuvantien.

Die Erfindung betrifft weiter die Verwendung mindestens einer Verbindung einer der allgemeinen Formeln C1 bis C16 oder mindestens einer der vorgenannten pharmazeutischen oder kosmetischen Zusammensetzungen zur Hemmung sowohl der Aktivität der Alanyl-Aminopeptidasen oder analoger Enzyme als auch der Aktivität der Dipeptidylpeptidase IV oder analoger Enzyme, und zwar allein oder in Kombination mit anderen Inhibitoren der Alanyl-Aminopeptidasen oder analoger Enzyme und/oder anderen Inhibitoren der DPIV oder analoger Enzyme.

Die Erfindung betrifft weiter die Verwendung mindestens einer Verbindung einer der allgemeinen Formeln C1 bis C16 oder mindestens einer der vorgenannten pharmazeutischen oder kosmetischen Zusammensetzungen zur topischen Beeinflussung sowohl der Aktivität der Alanyl-Aminopeptidasen oder analoger Enzyme als auch der Aktivität der Dipeptidylpeptidase IV oder analoger Enzyme, und zwar allein oder in Kombination mit anderen Inhibitoren der Alanyl-Aminopeptidasen oder analoger Enzyme und/oder anderen Inhibitoren der DPIV oder analoger Enzyme.

Die Erfindung betrifft weiter die Verwendung mindestens einer Verbindung einer der allgemeinen Formeln C1 bis C16 oder mindestens einer der vorgenannten pharmazeutischen oder gegebenenfalls auch kosmetischen Zusammensetzungen zur Prophylaxe und Therapie einer ganzen Anzahl von Erkrankungen, die in den Ansprüchen 35 bis 47 beispielhaft beansprucht sind. In besonderen Ausführungsformen, jedoch nicht beschränkend, können erfindungsgemäß die Verbindungen der allgemeinen Formeln C1 bis C16, insbesondere die in Table 1 bis 16 aufgeführten, besonders bevorzugten Einzelverbindungen C1.001 bis C16.013, als solche oder als Ausgangsstoffe für weitere Substanzen und in Kombination mit Inhibitoren der DPIV und analoger Enzyme und/oder in Kombination mit Inhibitoren der Alanyl-Aminopeptidasen zur Therapie von Erkrankungen mit überschießender Immunantwort (Autoimmunerkrankungen, Allergien und Transplantatrejektionen), von anderen chronisch-entzündlichen Erkrankungen, neuronalen Erkrankungen und zerebralen Schädigungen, Hauterkrankungen (u. a. Akne und Schuppenflechte), Tumorerkrankungen und speziellen Virusinfektionen (u. a. SARS) genutzt werden.

Die Erfindung betrifft weiter die Verwendung mindestens einer Verbindung einer der allgemeinen Formeln C1 bis C16 oder mindestens einer der vorgenannten pharmazeutischen oder kosmetischen Zusammensetzungen zur Herstellung eines Arzneimittels zur Hemmung sowohl der Aktivität der Alanyl-Aminopeptidasen oder analoger Enzyme als auch der Aktivität der Dipeptidylpeptidase IV oder analoger Enzyme, und zwar allein oder in Kombination mit anderen Inhibitoren der Alanyl-Aminopeptidasen oder analoger Enzyme und/oder anderen Inhibitoren der DPIV oder analoger Enzyme.

Die Erfindung betrifft weiter die Verwendung mindestens einer Verbindung einer der allgemeinen Formeln C1 bis C16 oder mindestens einer der vorgenannten pharmazeutischen oder kosmetischen Zusammensetzungen zur Herstellung eines Arzneimittels zur topischen Beeinflussung sowohl der Aktivität der Alanyl-Aminopeptidasen oder analoger Enzyme als auch der Aktivität der Dipeptidylpeptidase IV oder analoger Enzyme, und zwar allein oder in Kombination mit anderen Inhibitoren der Alanyl-Aminopeptidasen oder analoger Enzyme und/oder anderen Inhibitoren der DPIV oder analoger Enzyme.

Die Erfindung betrifft weiter die Verwendung mindestens einer Verbindung einer der allgemeinen Formeln C1 bis C16 oder mindestens einer der vorgenannten pharmazeutischen oder gegebenenfalls auch kosmetischen Zusammensetzungen zur Herstellung eines Arzneimittels zur Prophylaxe und Therapie einer ganzen Anzahl von Erkrankungen, die in den Ansprüchen 50 bis 62 beispielhaft beansprucht sind. In besonderen Ausführungsformen, jedoch nicht beschränkend, können erfindungsgemäß die Verbindungen der allgemeinen Formeln C1 bis C16, insbesondere die in den Table 1 bis 16 aufgeführten, besonders bevorzugten Einzelverbindungen C1.001 bis C16.013, als solche oder als Ausgangsstoffe für weitere Substanzen und in Kombination mit Inhibitoren der DPIV und analoger Enzyme zur Herstellung eines Arzneimittels zur Therapie von Erkrankungen mit überschießender Immunantwort (Autoimmunerkrankungen, Allergien und Transplantatrejektionen), von anderen chronisch-entzündlichen Erkrankungen, neuronalen Erkrankungen und zerebralen Schädigungen, Hauterkrankungen (u. a. Akne und Schuppenflechte), Tumorerkrankungen und speziellen Virusinfektionen (u. a. SARS) genutzt werden.

Die Erfindung betrifft weiter ein Verfahren zur Hemmung sowohl der Aktivität der Alanylpeptidasen oder analoger Enzyme als auch der Aktivität der Dipeptidylpeptidase IV oder analoger Enzyme, und zwar allein oder in Kombination mit anderen Inhibitoren der Alanyl-Aminopeptidasen oder analoger Enzyme und/oder anderen Inhibitoren der DPIV oder analoger Enzyme, durch Verabreichung mindestens einer Verbindung der allgemeinen Formeln C1 bis C16 oder mindestens einer der obigen pharmazeutischen oder kosmetischen Zusammensetzungen in einer für die Hemmung der Enzymaktivität erforderlichen Menge.

Die Erfindung betrifft weiter ein Verfahren zur topischen Beeinflussung sowohl der Aktivität der Alanylpeptidasen oder analoger Enzyme als auch der Aktivität der Dipeptidylpeptidase IV oder analoger Enzyme, und zwar allein oder in Kombination mit anderen Inhibitoren der Alanyl-Aminopeptidasen oder analoger Enzyme und/oder anderen Inhibitoren der DPIV oder analoger Enzyme, durch Verabreichung mindestens einer Verbindung der allgemeinen Formeln C1 bis C16 oder mindestens einer der obigen pharmazeutischen oder kosmetischen Zusammensetzungen in einer für die Hemmung der Enzymaktivität erforderlichen Menge.

Die Erfindung betrifft weiter ein Verfahren zur Prophylaxe und/oder Therapie einer der in den Ansprüchen 65 bis 77 beanspruchten Erkrankungen bzw. Zuständen unter Hemmung sowohl der Aktivität der Alanylpeptidasen oder analoger Enzyme als auch der Aktivität der Dipeptidylpeptidase IV oder analoger Enzyme, und zwar allein oder in Kombination mit anderen Inhibitoren der Alanyl-Aminopeptidasen oder analoger Enzyme und/oder anderen Inhibitoren der DPIV oder analoger Enzyme, durch Verabreichung mindestens einer Verbindung der allgemeinen Formeln C1 bis C16 oder mindestens einer der obigen pharmazeutischen oder kosmetischen Zusammensetzungen in einer für die Prophylaxe oder Therapie erforderlichen Menge.

Der Begriff "analoge Enzyme", wie er in der vorliegenden Beschreibung und in den Patentansprüchen verwendet wird, bezieht sich auf Enzyme, die eine der membranständigen Alanyl-Aminopeptidase bzw. der Dipeptidylpeptidase IV analoge Enzymaktivität aufweisen, wie dies beispielsweise für die zytosolische Alanyl-Aminopeptidase (APN) bzw. für FAP/Saprase oder das Attractin (DPIV) gilt. Der Begriff ist in diesem Sinn auch in der o. g. Druckschrift "A. J. Barrett et al.: Handbook of Proteolytic Enzymes, Academic Press 1998" erläutert.

In den allgemeinen Formeln C1 bis C16, wie sie sich aus den Ansprüchen 1, 3, 5, 7, 9, 11, 13, 15, 17, 19, 21, 23, 25, 27, 29, 30a und 30b in allgemeiner Form ergeben, stehen die Reste Rn, also die Reste R1, R2, R3, R4, R5, R6, R7, R8, R9, R10, R11, R12 und R13 jeweils unabhängig voneinander für einen Rest, der gewählt ist aus der aus der Gruppe, die besteht aus Wasserstoff, unsubstituiertem oder substituiertem, geradkettigem oder verzweigtem C₁- bis C₁₂-Alkyl, C₂- bis C₁₂-Alkenyl und C₂- bis C₁₂-Alkinyl, Hydroxy, Thiol, C₁- bis C₁₂-Alkoxy, C₁-bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carb-onyl, unsubstituiertem oder substituiertem Thiocarbonyl und unsubstituiertem oder substituiertem Imino.

Im einzelnen bedeuten die Reste Rn in erfindungsgemäßen Ausführungsformen dann, wenn sie für unsubstituierte geradkettige oder verzweigte Alkyl-Gruppen mit 1 bis 12 C-Atomen stehen, in bevorzugten Ausführungsformen Methyl, Ethyl, n-Propyl, i-Propyl, n-Butyl, i-Butyl, sec-Butyl, tert-Butyl, n-Pentyl, i-Pentyl, sec-Pentyl, tert-Pentyl, n-Hexyl, i-Hexyl, 3-Methylpentyl, 2-Ethylbutyl, 2,2-Dimethylbutyl sowie für die Reste Heptyl, Octyl, Nonyl. Decyl, Undecyl und Dodecyl alle geradkettigen und verzweigten Isomere. Erfindungsgemäß besonders bevorzugt aus der vorgenannten Gruppe sind Alkyl-Gruppen mit 1 bis 6 C-Atomen; von diesen sind die Reste Methyl, Ethyl, n-Propyl, i-Propyl, n-Butyl, i-Butyl, sec-Butyl und tert-Butyl noch mehr bevorzugt.

In anderen erfindungsgemäßen Ausführungsformen bedeuten die Reste Rn dann, wenn sie für unsubstituierte geradkettige oder verzweigte Alkenylgruppen mit 2 bis 12 C-Atomen stehen, in bevorzugten Ausführungsformen Vinyl, Allyl, 1-Butenyl, 2-Butenyl, sowie für die Reste Pentenyl, Hexenyl, Heptenyl, Octenyl, Nonenyl, Decenyl, Undecenyl und Dodecenyl alle geradkettigen und verzweigten und hinsichtlich der Stellung der C=C-Doppelbindung denkbaren Reste. In weiteren erfindungsgemäßen Ausführungsformen können die Reste Rn auch für geradkettige und verzweigte Alkenylgruppen mit mehreren Doppelbindungen stehen. Bevorzugte Reste aus dieser Gruppe stellen die Butadienyl-Gruppe und die Isoprenyl-Gruppe dar. Erfindungsgemäß besonders bevorzugt aus der vorgenannten Gruppe sind AlkenylGruppen mit 2 bis 6 C-Atomen; von diesen sind die Reste Vinyl, Allyl, 1-Butenyl und 2-Butenyl noch mehr bevorzugt.

In anderen erfindungsgemäßen Ausführungsformen bedeuten die Reste Rn dann, wenn sie für unsubstituierte geradkettige oder verzweigte Alkinylgruppen mit 2 bis 12 C-Atomen stehen, in bevorzugten Ausführungsformen Ethinyl, Propinyl, 1-Butinyl, 2-Butinyl, sowie für die Reste Pentinyl, Hexinyl, Heptinyl, Octinyl, Noninyl, Decinyl, Undecinyl und Dodecinyl alle geradkettigen und verzweigten und hinsichtlich der Stellung der C≡C-Dreifachbindung denkbaren Reste. Erfindungsgemäß besonders bevorzugt aus der vorgenannten Gruppe sind Alkinyl-Gruppen mit 2 bis 6 C-Atomen; von diesen sind die Reste Ethinyl, Propinyl, 1-Butinyl und 2-Butinyl noch mehr bevorzugt.

Sowohl geradkettige als auch verzweigte Alkyl-, Alkenyl- oder Alkinyl-Reste können erfindungsgemäß in einer weiteren Ausführungsform substituiert sein. Die Substituenten können an beliebigen Positionen des aus Kohlenstoffatomen gebildeten Grundgerüsts stehen und können gewählt sein aus der Gruppe, die besteht aus Halogenatomen wie Fluor, Chlor, Brom und Iod, Alkylgruppen mit 1 bis 6 C-Atomen, Hydroxygruppen, Alkoxygruppen mit 1 bis 6 C-Atomen im Alkylrest, Thiogruppen und Alkylthiogruppen mit 1 bis 6 C-Atomen im Alkylrest und unsubstituierten oder mit einem oder zwei Alkylresten mit jeweils unabhängig voneinander 1 bis 6 C-Atomen substituierten Aminogruppen.

In weiteren Ausführungsformen der Erfindung bedeuten die Reste Rn in den allgemeinen Formeln C1 bis C16 C₁- bis C₁₂-Alkoxy-Reste oder C₁- bis C₁₂-Alkylthio-Reste. Für die C₁-bis C₁₂-Alkylgruppen dieser Alkoxy- bzw. Alkylthio-Reste gelten die vorstehend genannten Definitionen der geradkettigen und verzweigten Alkyl-Reste ebenfalls. Besonders bevorzugt sind geradkettige C₁- bis C₆-Alkoxy-Reste und geradkettige C₁- bis C₆-Alkylthio-Reste, und besonders bevorzugt sind die Reste Methoxy, Ethoxy, n-Propoxy, Methylthio, Ethylthio und n-Propylthio.

In weiteren Ausführungsformen der Erfindung können die Reste Rn der allgemeinen Formeln C1 bis C16 auch stehen für unsubstituierte oder substituierte Cycloalkyl-Reste. Diese können erfindungsgemäß bevorzugt drei bis acht Atome im Ring enthalten und können entweder ausschließlich aus Kohlenstoff-Atomen bestehen oder ein oder mehrere Heteroatome enthalten. Besonders bevorzugt unter den rein carbocyclischen Ringen sind die Reste Cyclopentyl, Cyclopentenyl, Cyclopentadienyl, Cyclohexyl, Cyclohexenyl, Cyclohexadienyl, Cycloheptyl, Cycloheptenyl, Cycloheptadienyl und Cycloheptatrienyl; Beispiele für Heteroatome enthaltende Cycloalkyl-Reste sind in weiteren Ausführungsformen der Erfindung die Reste Tetrahydrofuranyl, Pyrrolidinyl, Pyrazolidinyl, Imidazolidinyl, Piperidinyl, Piperazinyl und Morpholinyl. Mögliche Substituenten an diesen carbocyclischen oder heterocyclischen Cycloalkylresten können gewählt sein aus der obigen Gruppe von Substituenten für lineare Alkyl-Gruppen.

In weiteren Ausführungsformen der Erfindung können die Reste Rn an den Verbindungen der allgemeinen Formeln C1 bis C16 stehen für unkondensierte oder kondensierte gegebenenfalls ein oder mehrere Heteroatome aus der Gruppe N, O, P und S enthaltende Aryl-Reste. Die Aryl-Reste können aus einem oder mehreren Ringen, bei mehreren Ringen bevorzugt aus zwei Ringen, bestehen; ein Ring kann weiter bevorzugt fünf, sechs oder sieben Ringglieder aufweisen. Bei aus mehreren aneinander kondensierten Ringen bestehenden Systemen sind Benzokondensierte Ringe besonders bevorzugt, d. h. Ringsysteme, in denen zumindest einer der Ringe ein aromatischer Sechsring ist. Besonders bevorzugt sind die rein aus Kohlenstoff-Atomen bestehenden Aryl-Reste gewählt aus Phenyl und Naphthyl; besonders bevorzugte Heteroatome enthaltende Aryl-Reste sind beispielsweise gewählt aus Indolyl, Cumaronyl, Thionaphthenyl, Chinolinyl (Benzopyridyl), Chinazolinyl (Benzopyrimidinyl) und Chinoxylinyl (Benzopyrazinyl).

Sowohl aus einem Ring bestehende als auch aus mehreren Ringen bestehende, sowohl nur Kohlenstoffatome enthaltende wie auch Heteroatome enthaltende aromatische und nichtaromatische cyclische Reste können erfindungsgemäß in einer weiteren Ausführungsform substituiert sein. Die Substituenten können an beliebigen Positionen des Ringsystems, sowohl an den Kohlenstoffatomen als auch an den Heteroatomen stehen und können beispielsweise gewählt sein aus der Gruppe, die besteht aus Halogenatomen wie Fluor, Chlor, Brom und Iod, Alkylgruppen mit 1 bis 6 C-Atomen, Alk-oxygruppen mit 1 bis 6 C-Atomen im Alkylrest und unsubstituierten oder mit einem oder zwei Alkylresten mit jeweils unabhängig voneinander 1 bis 6 C-Atomen substituierten Aminogruppen sowie mit Hydroxy-, Thio-, Ether- und Thioethergruppen.

Die Reste Rn (= R1 bis R13) können erfindungsgemäß weiter auch für unsubstituierte Amino-Reste (-NH₂) oder unsubstituierte Imino-Reste (-NH-) oder für substituierte Amino-Reste (-NHR1 oder -NR1Rm) oder substituierte Imino-Reste (-NRm) sowie Hydroxyreste OH, Alkoxy ORI, Thioreste SH und Alkylthioreste SRI stehen. Darin haben die Substituenten R1 und Rm die oben im einzelnen für die Reste Rn definierten Bedeutungen und können gleich oder verschieden sein.

Die Reste Rn (=R1 bis R13) können erfindungsgemäß weiter auch für unsubstituierte und substituierte, ein Phosphoratom enthaltende Reste stehen, beispielsweise für PH₂, PHRk, PRkRl, P(OH)₂, P(OH(ORk), P(ORk)(ORI), P(O)H₂, P(O)HRk, P(O)RkRl; oder können auch stehen für unsubstituierte und substituierte Oximreste =NOH, =NORk, sowie Hydroxylaminoreste NH(OH) oder Alkoxyaminoreste NH(ORk). Darin haben die Substituenten Rk und R1 die oben im einzelnen für die Reste R1 bis R13 definierten Bedeutungen und können gleich oder verschieden sein.

Die Reste Rn (= R1 bis R13) können erfindungsgemäß weiter auch für unsubstituierte Carbonyl-Reste (H-(C=O)-) oder unsubstituierte Thiocarbonyl-Reste (H-(C=S)-) oder für substituierte Carbonyl-Reste (Rm-(C=O)-) oder substituierte Thiocarbonyl-Reste (Rm-(C=S)-) stehen. Darin haben die Substitenten Rm substituierter Carbonyl-Reste oder substituierter Thiocarbonyl-Reste die oben im einzelnen für die möglichen Substituenten der Reste Rn definierten Bedeutungen.

In den Verbindungen der allgemeinen Formel C16 stehen die Reste Rn (=R1 bis R13) erfindungsgemäss für Wasserstoff, CH₃, CH₂Rk, CHRkR1, CRkR1Rm OH, ORk, NH₂, NHRk, NRkRl, C(O)Rk, C(NH)Rk, C(NRI)Rk, C(S)Rk, PH₂, PHRk, PRkRl, P(O)(OH)₂, P(O)(OH)(ORk), P(O)(ORk)(ORl), P(OH)₃, P(OH)₂ORk, P(OH)(ORk)(ORl), P(ORk)(ORl)(ORm), SH, SRk und CN, wobei die Reste Rk, Rl, Rm die bisher definierte Bedeutung der Reste R1 bis R13 haben.

Erfindungsgemäß können die vorgenannten Reste Rn (= R1, R2, R3, R4, R5, R6, R7, R8, R9, R10, R11, R12 und/oder R13) mit den jeweiligen Grundstrukturen der allgemeinen Formeln C1 bis C16 über eines ihrer Kohlenstoffatome verbunden sein. Es ist jedoch in einer alternativen Ausführungsform genauso gut möglich, daß die Reste Rn mit den jeweiligen Grundstrukturen der allgemeinen Formeln C1 bis C16 über das Heteroatom oder eines ihrer Heteroatome verbunden sind.

In mehreren der allgemeinen Formeln C1 bis C16 (beispielsweise in den allgemeinen Formeln C1, C6, C7, C12, C13, C 14) stehen Y, Y1 und Y2 für Reste, die über eine C=Y-Doppelbindung (bzw. C=Y1-Doppelbindung und/oder C=Y2-Doppelbindung) mit der Grundstruktur der jeweiligen Formel verbunden sind. Die Reste Y stehen in den allgemeinen Formeln, in denen sie vorkommen, jeweils unabhängig voneinander für einen der über eine Doppelbindung an ein Kohlenstoffatom gebundenen Reste O, S oder NRn, beispielsweise NR3 oder NR4 oder NR5, wobei in letzteren die Reste Rn (beispielsweise R3 oder R4 oder R5) die oben genannten Bedeutungen haben können, einschließlich der Bedeutung Wasserstoff. Besonders bevorzugt steht Y für über eine Doppelbindung an ein C-Atom gebundenes O.

In mehreren der allgemeinen Formeln C1 bis C16 (beispielsweise in den allgemeinen Formeln C8, C9, C10, C15) stehen X, X1, X2 und Z für Reste, die über je eine C-X-Einfachbindung (bzw. C-X1-Einfachbindung oder C-X2-Einfachbindung) oder eine C-Z-Einfachbindung an zwei verschiedene Kohlenstoffatome gebunden sind. Die Reste X und Z stehen in den allgemeinen Formeln, in denen sie vorkommen, jeweils unabhängig voneinander für einen der über je eine Einfachbindung an zwei verschiedene Kohlenstoffatome gebundenen Reste >NH, >NRn (z. B. >NR5 oder >NR10), -O-, -S-, -CH₂-, -CHRn- oder -CRn₂-, worin die Reste Rn die oben angegebene Bedeutung haben, oder stehen für einen der über je eine Einfachbindung an drei verschiedene Kohlenstoffatome gebundenen Reste >N-, >CH- oder >CRn- (z. B. >CR8- oder >CR9-), worin Rn (z. B. R8, R9) die oben angegebenen Bedeutungen haben.

In den Verbindungen der allgemeinen Formeln C1, C6, C7, C13 stehen X und Z unabhängig voneinander für Reste aus der Gruppe, die besteht aus Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl und Amino (NH₂, NHR1, NR1R2), worin alle vorgenannten Bedeutungen von X und Z denjenigen für Alkoxy, Alkylthio, Aryl, Cycloalkyl und Amino entsprechen, die oben für die Reste Rn der allgemeinen Formeln C1 bis C16 im einzelnen definiert wurden.

In den Verbindungen der allgemeinen Formel C10 stehen X und Z unabhängig voneinander für >CH-, >CR1- oder N; dabei stellt mindestens eine der beiden Gruppen ein Heteroatom der Grundstrukur dar oder besitzt ein solches; darin hat R1 die oben angegebenen Bedeutungen.

In den Verbindungen der allgemeinen Formel C4 stehen X1, X2, X3 und X4, die gleich oder verschieden sein können, für die Gruppe >CH- oder die Gruppe >CR3-, worin R3 die oben angegebenen Bedeutungen haben kann, und Y1, Y2 und Y3, die gleich oder verschieden sein können, stehen für nicht-substituierte oder substitierte Kohlenstoff-Einheiten oder Heteroatom-Einheiten mit den Ringatomen N, O, P oder S.

In den Verbindungen der allgemeinen Formel C16 sind A für Ausführungsbeispiele 30a und 30b sowie X, Y und Z für Ausführungsbeispiel 30a erfindungsgemäss jeweils ein unabhängig gewähltes Ringglied oder Teil eines Ringgliedes in einem homo- oder heterocyclischen kondensierten System, bestehend aus einem Fünf- und einem Sechsring. A ist Teil des Sechsringes, Y und Z sind Teil des Fünfringes. X ist Teil der Verknüpfung des kondensierten Systems.

A, Y und Z sind gewählt aus der Gruppe bestehend aus CH₂, CHRk, CRkRl, C(O), C(S), C(NH), C(NRk), NH, NRk, =NOH, =NORk, O, S, SO₂, PH, PRk, P(O)OH, P(O)ORk, P(OH)₃, P(OH)₂ORk, P(OH)(ORk)(ORI), P(ORk)(ORI)(ORm).

X steht für N, CH, CRk, P, P=O, P(OH)₂, P(OH)(ORk) oder P(ORk)(ORl).

Die Reste Rk, Rl und Rm haben die oben genannten Bedeutungen der Reste Rn (=R1 bis R13).

Die Verbindungen der in den Ansprüchen 1, 3, 5, 7, 9, 11, 13, 15, 17, 19, 21, 23, 25, 27, 29, 30a und 30b definierten allgemeinen Formeln C1 bis C16 im allgemeinen und die Verbindungen C1.001 bis C16.013 in Table 1 bis 16 in den Ansprüchen 2, 4, 6, 8, 10, 12, 14, 16, 18, 20, 22, 24, 26, 28, 30 und 30c im besonderen können nach an sich aus der Literatur bekannten Verfahren hergestellt werden bzw. sind kommerziell erhältlich.

Beansprucht werden die den allgemeinen Formeln C1 bis C16 entsprechenden Verbindungen im allgemeinen und die in Table 1 bis 16 genannten speziellen Verbindungen C1.001 bis C16.013 in bevorzugten Ausführungsformen der Erfindung zur Verwendung in der Medizin. Der Begriff "zur Verwendung in der Medizin" wird hier wie in den Patentansprüchen in seiner breitesten Bedeutung verstanden und bezieht sich auf alle denkbaren Anwendungsgebiete, in denen die durch die vorliegende Erfindung definierten Verbindungen der allgemeinen Formeln C1 bis C16, und in bevorzugten Ausführungsformen die Verbindungen C1.001 bis C16.013, wie sie speziell in Table 1 bis 16 aufgeführt sind, Wirksamkeit im Zusammenhang mit medizinisch relevanten Zuständen des Säugerkörpers, insbesondere des menschlichen Körpers, entfalten können.

Im Zusammenhang mit solchen medizinisch relevanten Zuständen findet eine Verwendung der Verbindungen der allgemeinen Formeln C1 bis C16 in allgemeinen und eine Verwendung der bevorzugten Verbindungen C1.001 bis C16.013 gemäß Table 1 bis 16 entweder in Form der Verwendung einer Einzelverbindung oder in Form der Verwendung mehrerer Verbindungen der allgemeinen Formeln C1 bis C16 (insbesondere der bevorzugten Verbindungen C1.001 bis C16.013 gemäß Table 1 bis 16) statt. Ebenfalls im Rahmen der Erfindung liegt eine Verwendung einer oder mehrerer der Verbindungen der allgemeinen Formeln C1 bis C16, bevorzugt einer oder mehrerer Verbindungen aus der Gruppe, die gewählt ist aus den Verbindungen C1.001 bis C16.013 gemäß Table 1 bis 16, in Kombination mit anderen Wirkstoffen, beispielsweise mit einer oder mehreren Verbindungen, die Wirksamkeit in der Inhibition von Alanyl-Aminopeptidasen oder von analogen Enzymen (also Enzymen mit gleicher Substratspezifität) und/oder Wirksamkeit in der Inhibition der Dipeptidylpeptidase IV (DPIV) oder von analogen Enzymen (also Enzymen mit gleicher Substratspezifität), aufwiesen. Beispiele solcher als Enzyminhibitor wirksamen Verbindungen werden in am gleichen Anmeldetag wie die vorliegende Anmeldung eingereichten parallelen Anmeldungen derselben Anmelder und in den eingangs zitierten Anmeldungen der Anmelder genannt, die durch die Inbezugnahme hinsichtlich ihres Offenbarungsgehalts in die vorliegende Beschreibung übernommen werden.

Spezielle Beispiele von als Inhibitor der Dipeptidylpeptidase IV oder analoger Enzyme wirksamen Inhibitoren, wie sie aus dem Stand der Technik bekannt sind und gegebenenfalls zusammen mit den Verbindungen gemäß der vorliegenden Erfindung, insbesondere mit einer oder mehreren der Verbindungen C1.001 bis C16.013 gemäß Table 1 bis 16, verwendet werden können, schließen beispielsweise ein: Xaa-Pro-Dipeptide, entsprechende Derivate, vorzugsweise Di-petidphosphonsäurediarylester, Dipeptidboronsäuren (z. B. Pro-boro-Pro) und deren Salze, Xaa-Xaa-(Trp)-Pro-(Xaa)n-Peptide (n = 0 bis 10), entsprechende Derivate und deren Salze bzw. Aminosäure (Xaa)-amide, entsprechende Derivate und deren Salze, wobei Xaa eine α-Aminosäure/Iminosäure bzw. ein α-Aminosäurederivat/Iminosäurederivat, vorzugsweise N^{ε}-4-Nitrobenzyl-oxycarbonyl-L-Lysin, L-Prolin, L-Tryptophan, L-Isoleucin, L-Valin ist und als Amidstruktur cyclische Amine, z.B. Pyrrolidin, Piperidin, Thiazolidin und deren Derivate fungieren. Derartige Verbindungen und deren Herstellung wurden in einem früheren Patent beschrieben (K. Neubert et al. DD296075A5). Weiter können als Effektoren für die DP IV zusammen mit den Verbindungen der allgemeinen Formeln C1 bis C16 gemäß der vorliegenden Erfindung mit Vorteil Tryptophan-1,2,3,4-tetrahydroisochinolin-3-carbonsäure-derivate (TSL) und (2S,2S',2S'')-2-[2'-[2''-amino-3''-(indol-3'''-yl)-1''-oxoprolyl]-1',2',3',4'-tetrahydro-6'8'-dihydroxy-7-methoxyisochinol-3-yl-carbonyl-amino]-4-hydrome-thyl-5-hydropentansäure (TMC-2A) verwendet werden. Ein beispielhafter, mit Vorteil zusammen mit den Verbindungen der allgemeinen Formeln D1 bis D14 verwendbarer Inhibitor von DP IV ist Lys[Z(NO₂]-thiazolidid, worin Lys für einen L-Lysin-Rest steht und Z(NO₂) für 4-Nitrobenzyloxycarbonyl steht (vgl. DD-A 296075).

Spezielle Beispiele von als Inhibitor der Alanylaminopeptidase wirksamen Inhibitoren, wie sie aus dem Stand der Technik bekannt sind und gegebenenfalls zusammen mit den Verbindungen gemäß der vorliegenden Erfindung, insbesondere mit einer oder mehreren der Verbindungen C1.001 bis C16.013 gemäß Table 1 bis 16, verwendet werden können, schließen beispielsweise ein: Actinonin, Leuhistin, Phebestin, Amastatin, Bestatin, Probestin, β-Aminothiole, α-Aminophosphinsäuren, α-Aminophosphinsäurederivate, vorzugsweise D-Phe-ψ-PO(OH)-CH₂]-Phe-Phe. Besonders bevorzugte bekannte und gemeinsam mit den Verbindungen gemäß der Erfindung zu verwendende Inhibitoren für die Alanyl-Amino-peptidase sind Bestatin (Ubenimex), Actinonin, Probestin, Phebestin, RB3014 oder Leuhistin.

Eine weitere Ausführungsform der Erfindung betrifft pharmazeutische Zubereitungen, die mindestens eine, gegebenenfalls auch zwei oder sogar noch mehr, Verbindung(en) der allgemeinen Formeln C1 bis C16, besonders bevorzugt ausgewählt aus den Verbindungen C1.001 bis C16.013 gemäß Table 1 bis 16, umfassen. Solche pharmazeutischen Zubereitungen umfassen eine oder mehrere der genannten Verbindungen jeweils in einer solchen Menge, wie sie zur Entfaltung einer pharmazeutischen Wirkung erforderlich ist. Solche Mengen kann der Fachmann im einzelnen anhand von wenigen Routinetests leicht und ohne erfinderisches Zutun ermitteln; sie liegen im allgemeinen in Bereichen von 0,01 bis 1000 mg jeder der Verbindungen der allgemeinen Formeln C1 bis C16, besonders bevorzugt der Verbindungen C1.001 bis C16.013 gemäß Table 1 bis 16, pro Darreichungseinheit, noch weiter bevorzugt in Bereichen von 0,1 bis 100 mg jeder der genannten Verbindungen pro Darreichungseinheit. Auf den jeweiligen einzelnen Säugerorganismus bzw. menschlichen Organismus abgestimmte Mengen kann darüber hinaus der Fachmann leicht ermitteln und gegebenenfalls auch vorsehen, daß eine ausreichende Konzentration der zu verwenden Verbindun(en) durch Darreichung geteilter oder mehrerer Darreichungsformen erreicht wird.

Eine weitere Ausführungsform der Erfindung betrifft kosmetische Zubereitungen, die mindestens eine, gegebenenfalls auch zwei oder sogar noch mehr, Verbindung(en) der allgemeinen Formeln C1 bis C16, besonders bevorzugt ausgewählt aus den Verbindungen C1.001 bis C16.013 gemäß Table 1 bis 16, umfassen. Solche kosmetischen Zubereitungen umfassen eine oder mehrere der genannten Verbindungen jeweils in einer solchen Menge, wie sie zur Entfaltung einer gewünschten, beispielsweise kosmetischen Wirkung erforderlich ist. Solche Mengen kann der Fachmann im einzelnen anhand von wenigen Routinetests leicht und ohne erfinderisches Zutun ermitteln; sie liegen im allgemeinen in Bereichen von 0,01 bis 1000 mg jeder der Verbindungen der allgemeinen Formeln C1 bis C16, besonders bevorzugt der Verbindungen C1.001 bis C16.013 gemäß Table 1 bis 16, pro Darreichungseinheit, noch weiter bevorzugt in Bereichen von 0,1 bis 100 mg jeder der genannten Verbindungen pro Darreichungseinheit. Auf den jeweiligen einzelnen Säugerorganismus bzw. menschlichen Organismus abgestimmte Mengen kann darüber hinaus der Fachmann leicht ermitteln und gegebenenfalls auch vorsehen, daß eine ausreichende Konzentration der zu verwendenden Verbindung(en) durch Darreichung geteilter oder mehrerer Darreichungsformen erreicht wird.

Die eine oder mehreren Verbindungen gemäß der vorliegenden Erfindung oder diese enthaltende pharmazeutische oder kosmetische Zubereitungen werden simultan mit bekannten Trägerstoffen und/oder Hilfsstoffen (Adjuvantien) verabreicht. Solche Träger- und Hilfsstoffe sind dem Fachmann als solche und auch hinsichtlich ihrer Funktion und Anwendungsweise bekannt und bedürfen daher an dieser Stelle keiner detaillierten Erläuterung.

Von der Erfindung umfaßt sind auch pharmazeutische Zubereitungen, die umfassen: einen oder mehrere der Inhibitoren der DP IV bzw. der Inhibitoren von Enzymen mit DP IVanaloger Enzymaktivität oder/und der Inhibitoren der APN bzw. der Inhibitoren von Enzymen mit APN-analoger Enzymaktivität gemäß dem Stand der Technik, zusammen mit einer oder mehreren Verbindung(en) der allgemeinen Formeln C1 bis C16, insbesondere bevorzugt zusammen mit einer oder mehreren der Verbindungen, die aus den Verbindungen C1.001 bis C16.013 der Tablen 1 bis 15 ausgewählt sind, in räumlich getrennter Formulierung in Kombination mit an sich bekannten Träger-, Hilfs- und/oder Zusatzstoffen zur gleichzeitigen oder zeitlich unmittelbar aufeinanderfolgenden Verabreichung mit dem Ziel einer gemeinsamen Wirkung.

Die Verabreichung der Verbindungen der allgemeinen Formeln C1 bis C16 im allgemeinen und bevorzugt der Verbindungen C1.001 bis C16.013 gemäß Table 1 bis 16 bzw. pharmazeutischer oder kosmetischer Zubereitungen, die eine oder mehrere der vorgenannten Verbindungen zusammen mit an sich üblichen Träger-, Hilfs- und/oder Zusatzstoffen umfassen, erfolgt einerseits als topische Applikation in Form von z.B. Cremes, Salben, Pasten, Gelen, Lösungen, Sprays, Liposomen und Nanosomen, Schüttelmixturen, "pegylierten" Formu-lierungen, degradierbaren (d. h. unter physiologischen Bedingungen abbaubaren) Depot-Ma-trices, Hydrokolloidverbänden, Pflastern, Mikroschwämmen, Prepolyomeren und ähnlichen neuen Trägersubstraten, Jet-Injektion bzw. anderen dermatologischen Grundlagen/Vehikeln einschließlich instillativer Applikation, und andererseits als systemische Applikation zur oralen, transdermalen, intravenösen, subcutanen, intracutanen, intramuskulären, intrathekalen Anwendung in geeigneten Rezepturen bzw. in geeigneter Galenik.

Erfindungsgemäß werden die Verbindungen der allgemeinen Formeln C1 bis C16 im allgemeinen und bevorzugt die Verbindungen C1.001 bis C16.013 gemäß Table 1 bis 16 einzeln oder in Kombination, oder auch pharmazeutische oder kosmetische Zusammensetzungen, die eine oder mehrere der genannten Verbindungen umfassen, zur Hemmung sowohl der Aktivität der Alanyl-Aminopeptidasen oder analoger Enzyme als auch der Aktivität der Dipeptidylpeptidase IV oder analoger Enzyme, und zwar allein oder in Kombination mit anderen Inhibitoren der Alanyl-Aminopeptidasen oder analoger Enzyme und/oder anderen Inhibitoren der DPIV oder analoger Enzyme verwendet.

In einer weiteren Ausführungsform der Erfindung werden die Verbindungen der allgemeinen Formeln C1 bis C16 im allgemeinen und bevorzugt die Verbindungen C1.001 bis C16.013 gemäß Table 1 bis 16 einzeln oder in Kombination, oder auch pharmazeutische oder kosmetische Zusammensetzungen, die eine oder mehrere der genannten Verbindungen umfassen, zur topischen Beeinflussung sowohl der Aktivität der Alanyl-Aminopeptidasen oder analoger Enzyme als auch der Aktivität der Dipeptidylpeptidase IV oder analoger Enzyme, und zwar allein oder in Kombination mit anderen Inhibitoren der Alanyl-Aminopeptidasen oder analoger Enzyme und/oder anderen Inhibitoren der DPIV oder analoger Enzyme verwendet.

In bevorzugten Ausführungsformen der Erfindung erfolgt eine Verwendung der Verbindungen der allgemeinen Formeln C1 bis C16 im allgemeinen und bevorzugt der Verbindungen C1.001 bis C16.013 gemäß Table 1 bis 16 einzeln oder in Kombination, oder auch eine Verwendung von pharmazeutischen oder kosmetischen Zusammensetzungen, die eine oder mehrere der genannten Verbindungen umfassen, zur Prophylaxe und Therapie von Erkrankungen wie beispielsweise Multiple Sklerose, Morbus Crohn, Colitis ulcerosa, und anderen Autoimmunerkrankungen sowie entzündlichen Erkrankungen, Asthma bronchiale und anderen allergischen Erkrankungen, Haut- und Schleimhauterkrankungen, beispielsweise Psoriasis, Akne sowie dermatologischen Erkrankungen mit Hyperproliferation und veränderten Differenzierungszuständen von Fibroblasten, benigner fibrosierender und sklerosierender Hauterkrankungen und maligner fibroblastärer Hyperproliferationszustände, akuten neuronalen Erkrankungen, wie beispielsweise Ischämie-bedingter zerebraler Schädigungen nach einem ischämischen oder hämorrhagischen Schlaganfall, Schädel/Hirn-Trauma, Herzstillstand, Herzinfarkt oder als Folge von herzchirurgischen Eingriffen, von chronischen neuronalen Erkrankungen, beispielsweise von Morbus Alzheimer, der Pick'schen Erkrankung, der Progressiven Supranukleären Palsy, der kortikobasalen Degeneration, der frontotemporalen Demenz, von Morbus Parkinson, insbesondere Parkinsonismus gekoppelt an Chromosom 17, von Morbus Huntington, von durch Prionen bedingten Krankheitszuständen und von Amyotropher Lateralsklerose, von Artherosklerose, arterieller Entzündung, Stent-Restenose, von Chronisch Obstruktiven Lungenerkrankungen (COPD), von Tumoren, Metastasierungen, von Prostatakarzinom, von Schwerem Akutem Respiratorischen Syndrom (SARS) und von Sepsis und Sepsis-ähnlichen Zuständen.

In einer weiteren bevorzugten Ausführungsform der Erfindung erfolgt eine Verwendung der Verbindungen der allgemeinen Formeln C1 bis C16 im allgemeinen und bevorzugt die Verbindungen C1.001 bis C16.013 gemäß Table 1 bis 16 einzeln oder in Kombination, oder auch der pharmazeutischen oder kosmetischen Zusammensetzungen, die eine oder mehrere der genannten Verbindungen umfassen, zur Prophylaxe und Therapie der Abstoßung von transplantierten Geweben und Zellen. Als ein Beispiel einer solchen Anwendung kann die Verwendung einer oder mehrerer der vorgenannten Verbindungen oder einer pharmazeutischen Zusammensetzung, die eine oder mehrere der genannten Verbindungen enthält, bei allogenen oder xenogenen transplantierten Organen, Geweben und Zellen, wie Nieren-, Herz-, Leber-, Pankreas-, Haut- oder Stammzelltransplantation sowie Graft versus Host-Erkrankungen genannt werden.

In einer weiteren bevorzugten Ausführungsform der Erfindung erfolgt eine Verwendung der Verbindungen der allgemeinen Formeln C1 bis C16 im allgemeinen und bevorzugt der Verbindungen C1.001 bis C16.013 gemäß Table 1 bis 16 einzeln oder in Kombination, oder auch der pharmazeutischen oder kosmetischen Zusammensetzungen, die eine oder mehrere der genannten Verbindungen umfassen, zur Prophylaxe und Therapie der Abstoßungs- oder Entzündungsreaktionen an oder durch in einen Organismus implantierte medizinische Gegenstände ("medical devices"). Dies können beispielsweise Stents, Gelenkimplantate (Kniegelenk-Implantate, Hüftgelenk-Implantate), Knochen-Implantate, Herz-Schrittmacher oder andere Implantate sein. In einer weiteren bevorzugten Ausführungsform der Erfindung erfolgt eine Verwendung der Verbindungen der allgemeinen Formeln C1 bis C16 im allgemeinen und bevorzugt die Verbindungen C1.001 bis C16.013 gemäß Table 1 bis 16 einzeln oder in Kombination, oder auch der pharmazeutischen oder kosmetischen Zusammensetzungen, die eine oder mehrere der genannten Verbindungen umfassen, in der Weise, daß die Verbindung(en) oder Zusammensetzung(en) in Form einer Beschichtung oder Benetzung auf den Gegenstand bzw. die Gegenstände aufgebracht werden oder mindestens eine der Verbindungen oder Zusammensetzungen stofflich dem Material des Gegenstandes / der Gegenstände beigemischt wird. Auch in diesem Fall ist natürlich möglich, mindestens eine der Verbindungen oder Zusammensetzungen - gegebenenfalls zeitlich abgestuft oder parallel - lokal oder systemisch zu verabreichen.

In gleicher Weise wie vorstehend beschrieben - und für die vergleichbaren Zwecke bzw. zur Prophylaxe und Therapie der vorstehend beispielhaft, jedoch nicht abschließend genannten Erkrankungen und Zustände - können die Verbindungen der allgemeinen Formeln C1 bis C16 im allgemeinen und die Verbindungen C1.001 bis C16.013 gemäß Table 1 bis 16 in bevorzugten Ausführungsformen, sowie die vorstehend beschriebenen, die genannten Verbindungen enthaltenden pharmazeutischen und kosmetischen Zusammensetzungen allein oder in Kombination mehrerer von ihnen zur Herstellung von Medikamenten zur Behandlung der o. g. Krankheiten oder Zustände verwendet werden. Diese können die genannten Verbindungen in den vorstehend genannten Mengen umfassen, gegebenenfalls zusammen mit an sich bekannten Träger-, Hilfs- und/oder Zusatzstoffen.

Die Erfindung betrifft abschließend auch ein Verfahren zur Hemmung sowohl der Aktivität der Alanyl-Aminopeptidasen oder analoger Enzyme als auch der Aktivität der Dipeptidylpeptidase IV oder analoger Enzyme, und zwar allein oder in Kombination mit anderen Inhibitoren der Alanyl-Aminopeptidasen oder analoger Enzyme und/oder anderen Inhibitoren der DPIV oder analoger Enzyme durch Verabreichung mindestens einer Verbindung oder pharmazeutischen oder kosmetischen Zusammensetzung gemäß der obigen detaillierten Beschreibung in einer für die Hemmung der Enzymaktivität erforderlichen Menge. Die Mengen einer der Verbindungen der allgemeinen Formeln C1 bis C16 im allgemeinen bzw. der Verbindungen C1.001 bis C16.013 gemäß Table 1 bis 16 liegen - wie oben angegeben - im Bereich von 0,01 bis 1000 mg einer Verbindung pro Verabreichungseinheit, vorzugsweise im Bereich von 0,1 bis 100 mg pro Verabreichungseinheit.

Weiter betrifft die Erfindung auch ein Verfahren zur topischen Beeinflussung sowohl der Aktivität der Alanyl-Aminopeptidasen oder analoger Enzyme als auch der Aktivität der Dipeptidylpeptidase IV oder analoger Enzyme, und zwar allein oder in Kombination mit anderen Inhibitoren der Alanyl-Aminopeptidasen oder analoger Enzyme und/oder anderen Inhibitoren der DPIV oder analoger Enzyme durch Verabreichung mindestens einer Verbindung oder pharmazeutischen oder kosmetischen Zusammensetzung gemäß der vorstehenden detaillierten Beschreibung in einer für die Beeinflussung der Enzymaktivität erforderlichen Menge. Auch in diesen Fällen bewegen sich die Mengen der Verbindung(en) im oben angegebenen Bereich.

Weiter betrifft die Erfindung auch ein Verfahren zur Prophylaxe und Therapie einer Vielzahl von Erkrankungen, beispielsweise Erkrankungen mit überschießender Immunantwort (Autoimmunerkrankungen, Allergien und Transplantatrejektionen), von anderen chronisch-entzündlichen Erkrankungen, neuronalen Erkrankungen und zerebralen Schädigungen, Hauterkrankungen (u. a. Akne und Schuppenflechte), Tumorerkrankungen und speziellen Virusinfektionen (u. a. SARS) und insbesondere der oben im einzelnen genannten Erkrankungen, durch Verabreichung mindestens einer Verbindung oder pharmazeutischen oder kosmetischen Zusammensetzung gemäß der vorstehenden detaillierten Beschreibung in einer für die Prophylaxe oder Therapie der jeweiligen Erkrankung erforderlichen Menge. Auch in diesen Fällen bewegen sich die Mengen der Verbindung(en) im oben angegebenen Bereich von 0,01 bis 1000 mg einer Verbindung pro Verabreichungseinheit, vorzugsweise im Bereich von 0,1 bis 100 mg pro Verabreichungseinheit.

Die Erfindung wird nachfolgend durch spezielle bevorzugte Ausführungsbeispiele näher erläutert. Die nachfolgenden Ausführungsbeispiele dienen jedoch nicht der Beschränkung der Erfindung, sondern ausschließlich deren beispielhafter Erläuterung.

### Ausführungsbeispiele

### Beispiel 1:

### Inhibitonscharakteristika neuartiger dualer Hemmstoffe der Dipeptidylpeptidase IV und der Alanyl-Aminopeptidasen.

In den nachfolgen Table 1 bis 16 sind neue Hemmstoffe zusammengefasst für die durch die Anmelder gezeigt werden konnte, dass diese Substanzen in der Lage sind, beide Peptidasen und analog wirkende Enzyme gemeinsam in ihrer enzymatischen Aktivität zu inhibieren. Die gemessenen Inhibitionscharakteristika sind als IC-50- oder ID-50-Werte (letztere markiert mit "*") für beide Enzyme angegeben. Die enzymatische Aktivität wurde mit Hilfe der fluorogenen Substrate/Produkte (Ala-Pro)₂-Rhodamin 110 bzw. (Ala)₂-Rhodamin 110 ermittelt.

**Table 1:**

| **Compound ID.** | **Structure** | **IC50_{DPIV/APN} [**µ**M]** |
|---|---|---|
| C1.001 | 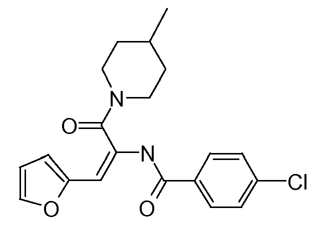 | 18.5 / 6.2 |
| C1.002 | 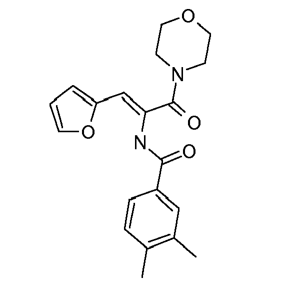 | 46.5 / 28.8 |
| C1.003 | 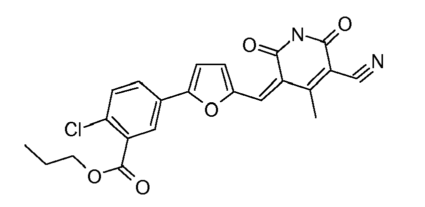 | 65.3 / 36.8 |

**Table 2:**

| **Compound ID.** | **Structure** | **IC50_{DPIV/APN} [**µ**M]** |
|---|---|---|
| C2.001 | 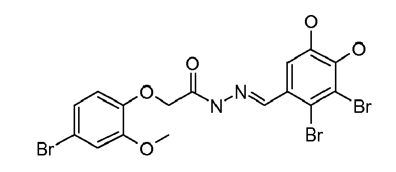 | 10.2 / 5.0 |
| C2.002 | 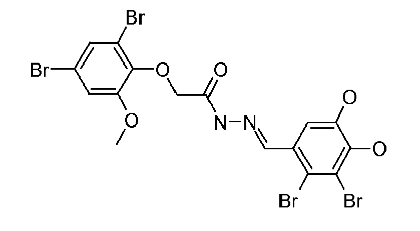 | 9.1 / 7.2 |
| C2.003 | 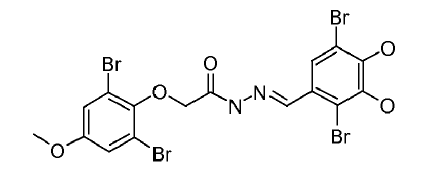 | 29.2 / 5.4* |
| C2.004 | 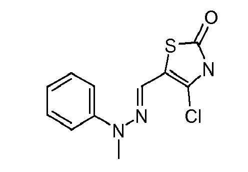 | 32.4 / 14.4 |
| C2.005 | 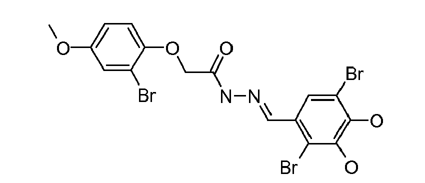 | 40.8 / 15.0 |
| C2.006 | 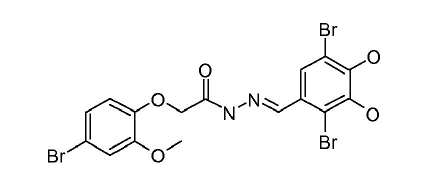 | 45.0 / 20.5 |
| C2.007 | 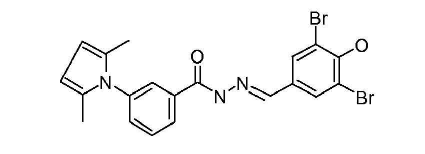 | 41.1 / 24.9 |
| C2.008 | 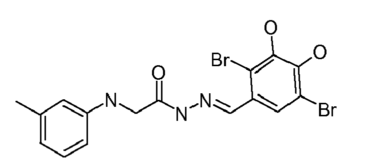 | 65.5* / 7.8 |
| C2.009 | 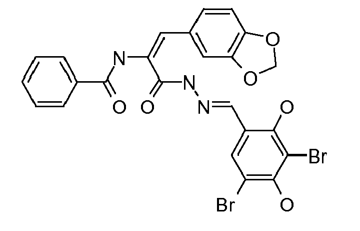 | 60.0 / 23.6 |
| C2.010 | 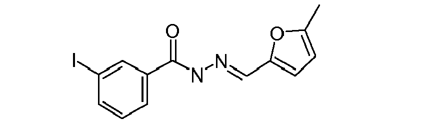 | 57.4 / 30.0 |
| C2.011 | 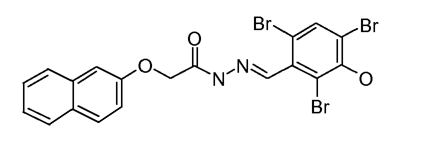 | 69.6 / 21.0 |
| C2.012 | 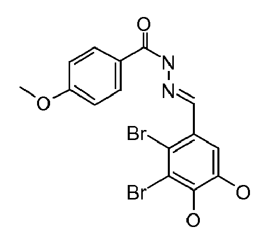 | 57.8 / 33.6* |
| C2.013 | 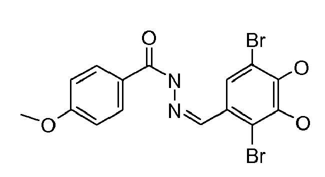 | 94.0* / 39.7* |
| C2.014 | 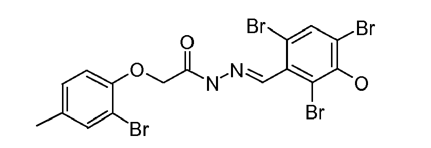 | 101.4* / 54.2* |
| C2.015 | 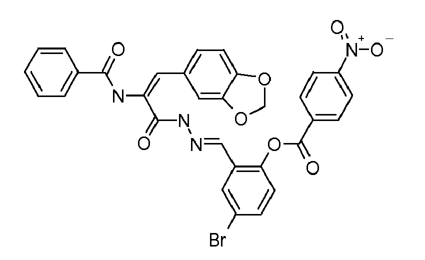 | 271.8* / 54.2 |

**Table 3:**

| **Compound ID.** | **Structure** | **IC50_{DPIV/APN} [**µ**M]** |
|---|---|---|
| C3.001 | 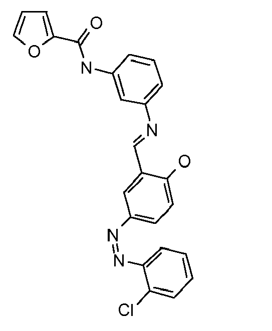 | 2.5* / 3.6 |
| C3.002 | 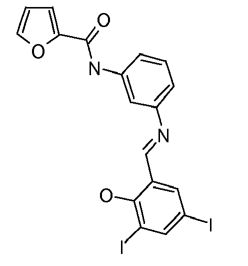 | 9.2 / 3.0 |
| C3.004 | 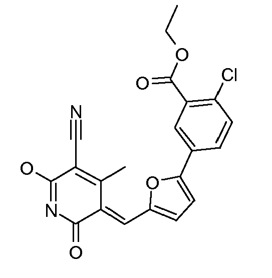 | 56.7 / 52.1 |

**Table 4:**

| **Compound ID.** | **Structure** | **IC50_{DPIV/APN} [**µ**M]** |
|---|---|---|
| C4.002 | 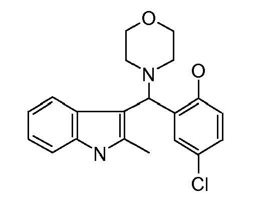 | 12.1 / 11.8 |
| C4.005 | 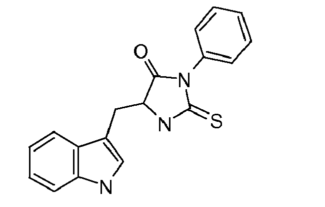 | 40.2 / 12.4 |
| C4.006 | 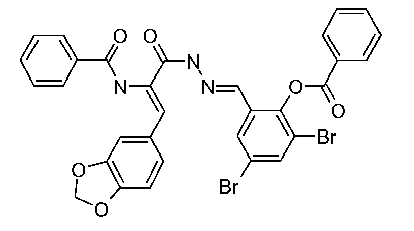 | 38.6 / 19.8 |
| C4.007 | 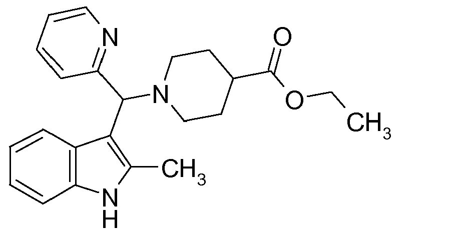 | 32.0 / 32.0 |
| C4.008 | 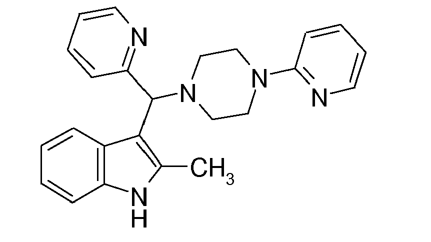 | 45.0 / 28.0 |
| C4.009 | 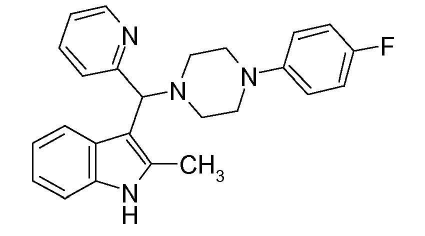 | 96.0 / 76.0 |
| C4.010 | 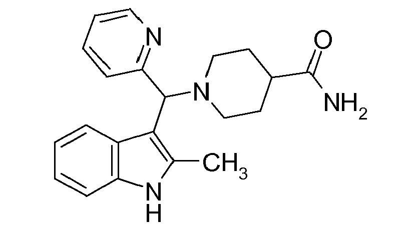 | 54.0 / 42.0 |
| C4.011 | 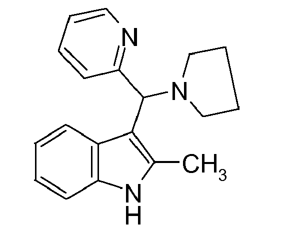 | 170.0 / 200.0 |
| C4.012 | 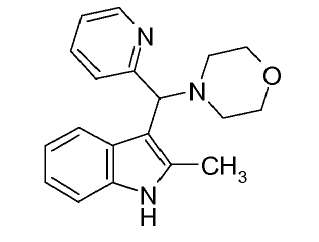 | 59.0 / 36.0 |
| C4.013 | 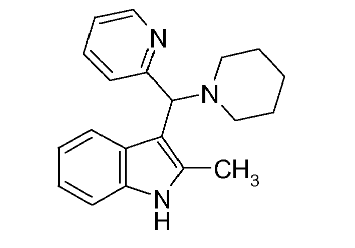 | 74.0 / 78.0 |
| C4.014 | 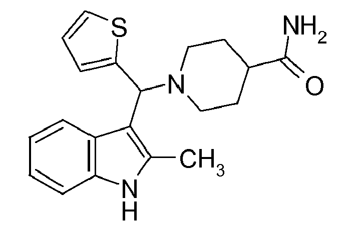 | 61.0 / 48.0 |
| C4.015 | 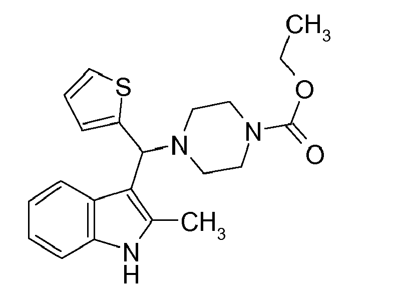 | 49.0 / 25.0 |
| C4.016 | 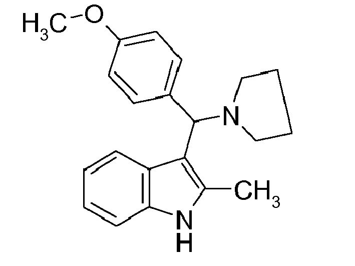 | 49.0 / 30.0 |
| C4.017 | 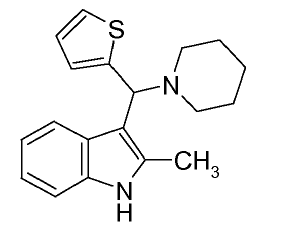 | 67.0 / 31.0 |
| C4.018 | 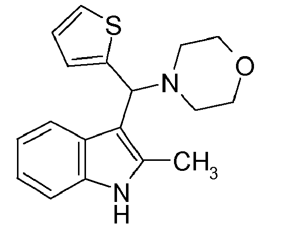 | 61.0 / 32.0 |
| C4.019 | 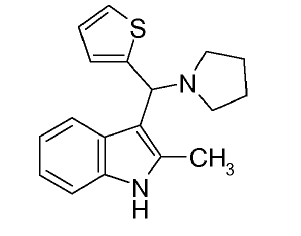 | 57.0 / 35.0 |

**Table 5:**

| **Compound ID.** | **Structure** | **IC50_{DPIV/APN} [**µ**M]** |
|---|---|---|
| C5.001 | 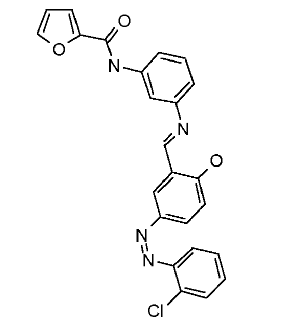 | 2.5* / 3.6 |
| C5.002 | 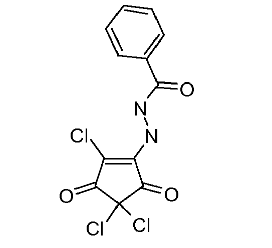 | 26.2 / 15.2 |

**Table 6:**

| **Compound ID.** | **Structure** | **IC50_{DPIV/APN} [**µ**M]** |
|---|---|---|
| C6.001 | 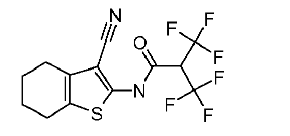 | 39.5 / 71.4 |
| C6.002 | 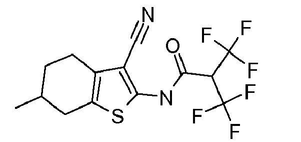 | 59.8 / 51.3 |

**Table 7:**

| **Compound ID.** | **Structure** | **IC50_{DPIV/APN} [**µ**M]** |
|---|---|---|
| C7.001 | 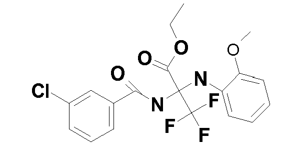 | 18.9 / 21.7 |
| C7.002 | 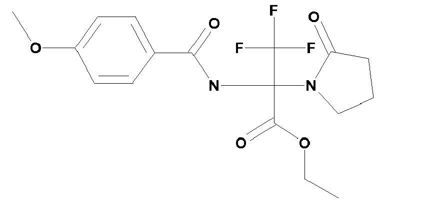 | 25.4 / 48.7 |
| C7.003 | 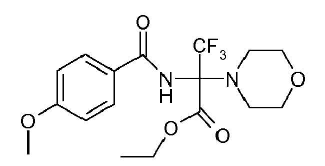 | 29.2 / 90.2 |
| C7.004 | 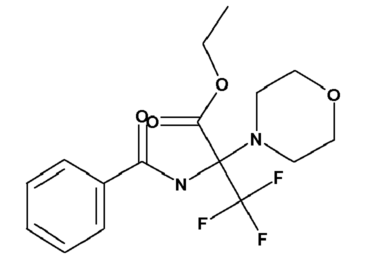 | 61.9 / 186.0* |

**Table 8:**

| **Compound ID** | **Structure** | **IC50_{DPIV/APN} [**µ**M]** |
|---|---|---|
| C8.001 | 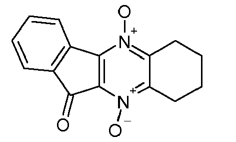 | 14.0 / 2.0* |
| C8.002 | 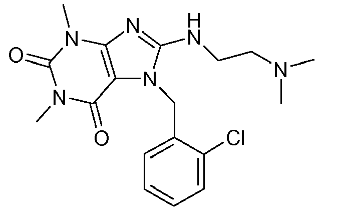 | 7.5 / Inactive |
| C8.003 | 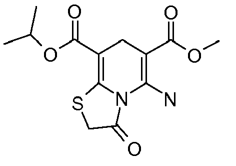 | 10.8 / 5.9 |
| C8.004 | 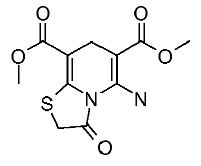 | 34.1 / very high positive* |
| C8.005 | 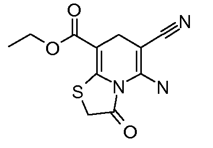 | 14.1 / 23.1 |
| C8.006 | 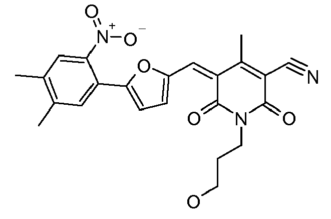 | 34.8 / 9.7 |
| C8.007 | 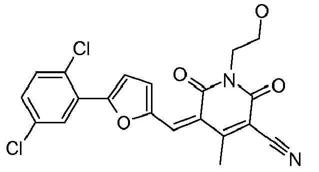 | 51.7 / 1.2* |
| C8.008 | 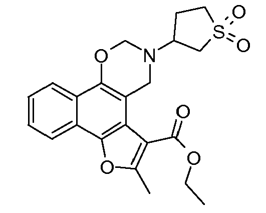 | 34.2 / 22.3 |
| C8.009 | 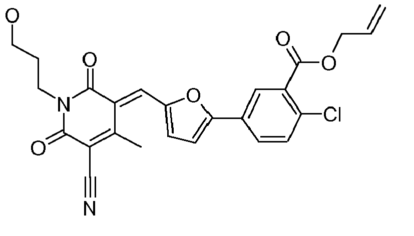 | 44.6 / 18.1 |
| C8.010 | 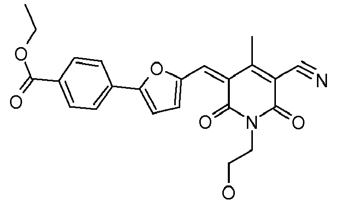 | 65.9 / 2.7* |
| C8.011 | 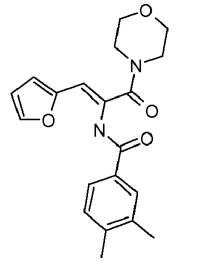 | 46.5 / 28.8 |
| C8.012 | 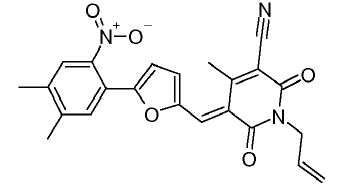 | 65.4 / 29.3 |
| C8.013 | 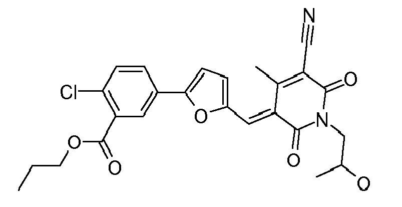 | 82.2 / 32.0 |
| C8.014 | 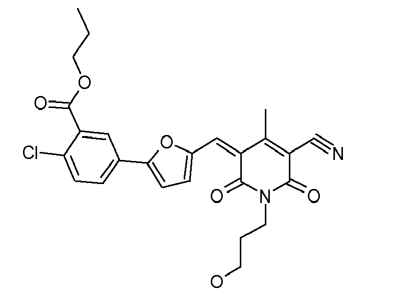 | 76.3 / 23.8 |
| C8.015 | 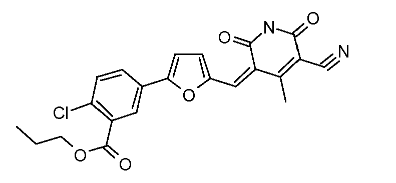 | 65.3 / 36.8 |
| C8.016 | 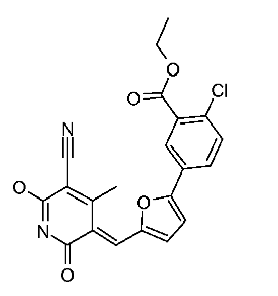 | 56.7 / 52.1 |
| C8.017 | 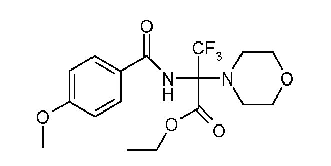 | 29.2 / 90.2 |
| C8.018 | 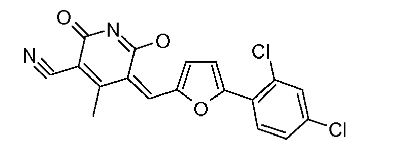 | 95.8 / 30.3 |
| C8.019 | 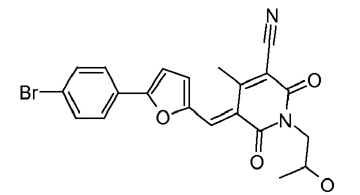 | 73.8 / 47.2* |
| C8.020 | 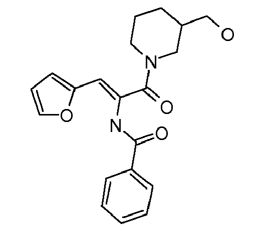 | 152.5* / 19.0 |
| C8.021 | 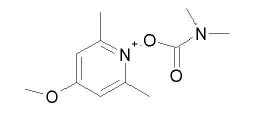 | 118.9* / 42.4 |
| C8.022 | 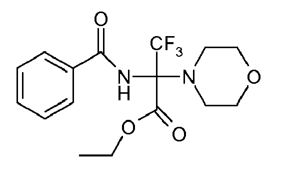 | 61.9 / 186.0* |
| C8.023 | 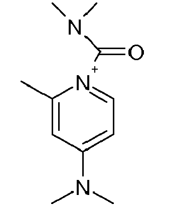 | 283.6* / 27.6 |

**Table 9:**

| **Compound ID.** | **Structure** | **IC50_{DPIV/APN} [**µ**M]** |
|---|---|---|
| C9.001 | 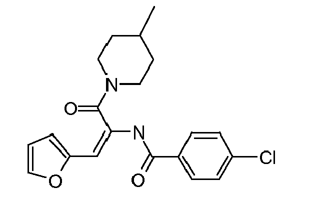 | 18.5 / 6.2 |
| C9.002 | 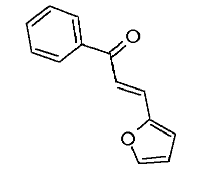 | 19.7 / 27.3 |
| C9.003 | 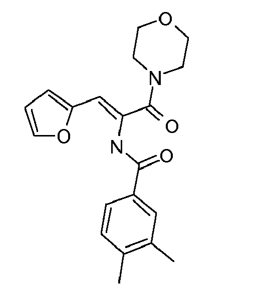 | 46.5 / 28.8 |
| C9.004 | 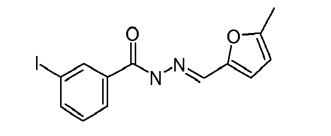 | 57.4 / 30.0 |
| C9.005 | 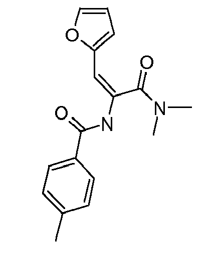 | 47.7 / 32.5 |
| C9.006 | 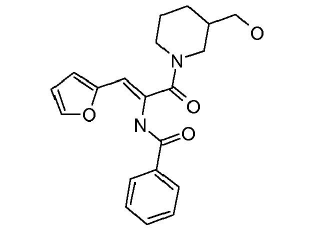 | 152.5 / 19.0 |

**Table 10:**

| **Compound ID.** | **Structure** | **IC50_{DPIV/APN} [**µ**M]** |
|---|---|---|
| C10.003 | 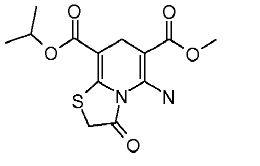 | 10.8 / 5.9 |
| C10.005 | 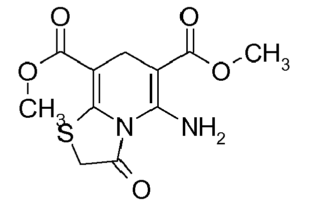 | 34.1 / very high positive* |
| C10.012 | 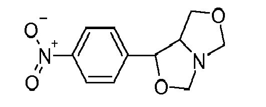 | 42.4 / 64.5 |
| C10.015 | 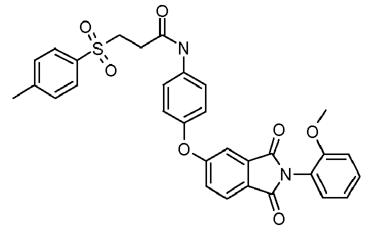 | 99.4 / 26.7 |

**Table 11:**

| **Compound ID.** | **Structure** | **IC50_{DPIV/APN} [**µ**M]** |
|---|---|---|
| C11.001 | 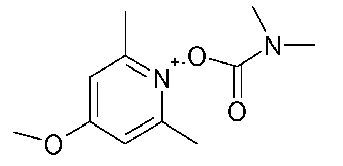 | 118.9* / 42.4 |
| C11.002 | 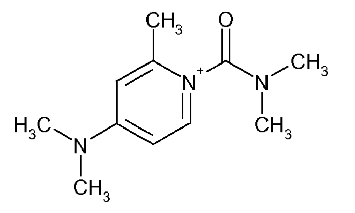 | 283.6* / 27.6 |

**Table 12:**

| **Compound ID.** | **Structure** | **IC50_{DPIV/APN} [**µ**M]** |
|---|---|---|
| C12.001 | 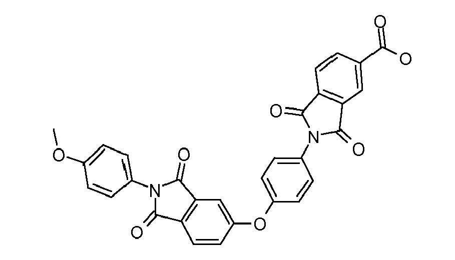 | 20.6 / 14.8 |

**Table 13:**

| **Compound ID.** | **Structure** | **IC50_{DPIV/APN} [**µ**M]** |
|---|---|---|
| C13.001 | 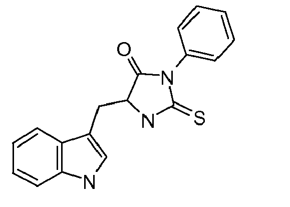 | 40.2 / 12.4 |
| C13.002 | 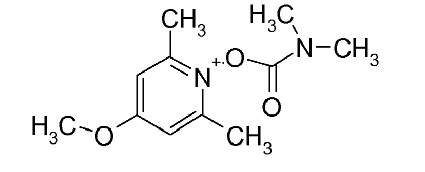 | 118.9* / 42.4 |
| C13.003 | 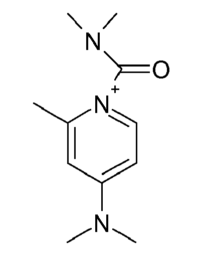 | 283.6* / 27.6 |

**Table 14:**

| **Compound ID.** | **Structure** | **IC50_{DPIV/APN} [**µ**M]** |
|---|---|---|
| C14.001 | 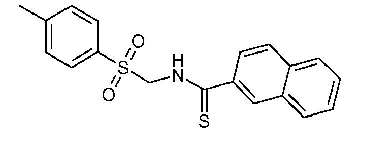 | 14.5 / 11.8 |
| C14.002 | 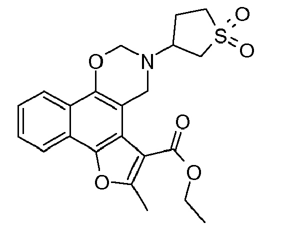 | 34.2 /22.3 |

**Table 15:**

| **Compound ID.** | **Structure** | **IC50_{DPIV/APN} [µM]** |
|---|---|---|
| C15.002 | 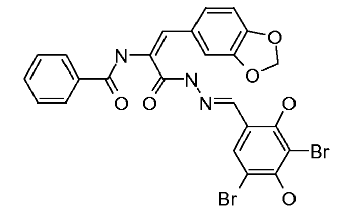 | 60.0 / 23.6 |
| C15.003 | 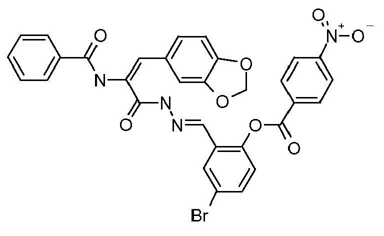 | 271.8* / 54.2 |
| C15.004 | 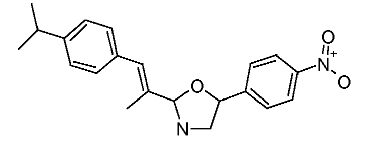 | 65.0 / 293.0* |

**Table 16**

| **Compound ID.** | **Structure** | **IC50_{DPIV/APN} [µM]** |
|---|---|---|
| C16.001 | 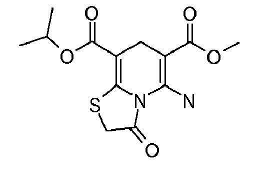 | 10.8 / 6.9 |
| C16.003 | 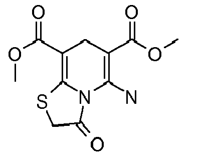 | 34.1 / very high positive** |
| C16.004 | 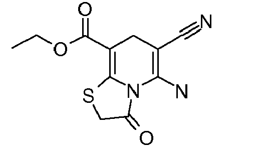 | 14.1 / 23.1 |
| C16.005 | 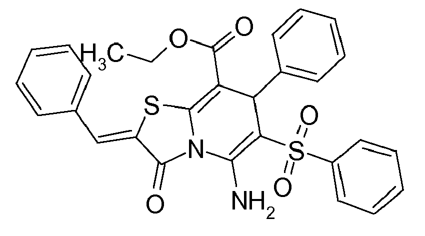 | 200.0 / 200.0 |
| C16.006 | 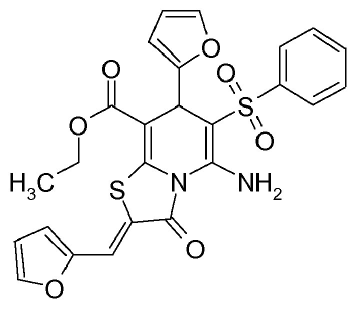 | 200.0 / 200.0 |
| C16.007 | 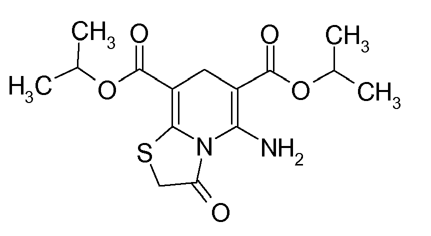 | 79.0 / 75.0 |
| C16.008 | 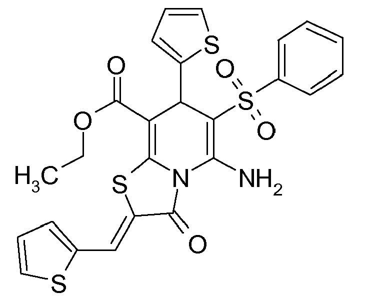 | 139.0 / 200.0 |
| C16.009 | 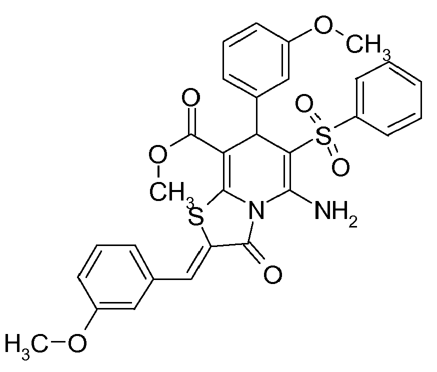 | 140.0 / 200.0 |
| C16.010 | 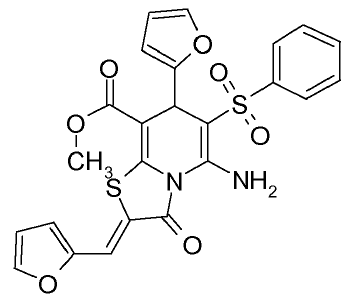 | 200.0 / 100.0 |
| C16.011 | 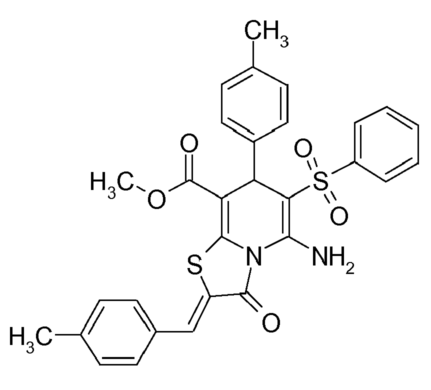 | 115.0 / 200.0 |
| C16.012 | 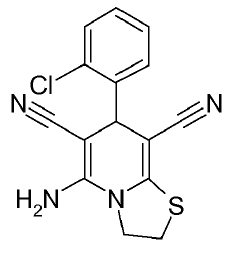 | 200.0 / 200.0 |
| C16.013 | 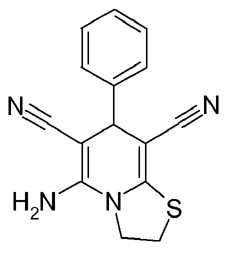 | 200.0 / 200.0 |

### Beispiel 2:

### Therapeutische Wirkung der kombinierten Hemmung der Alanyl-Aminopeptidasen und analog wirkender Enzyme sowie der Dipeptidylpeptidase IV und analog wirkender Enzyme auf die Experimentelle Autoimmune Enzephalomyelitis (EAE) der Maus als Tiermodell der Multiplen Sklerose

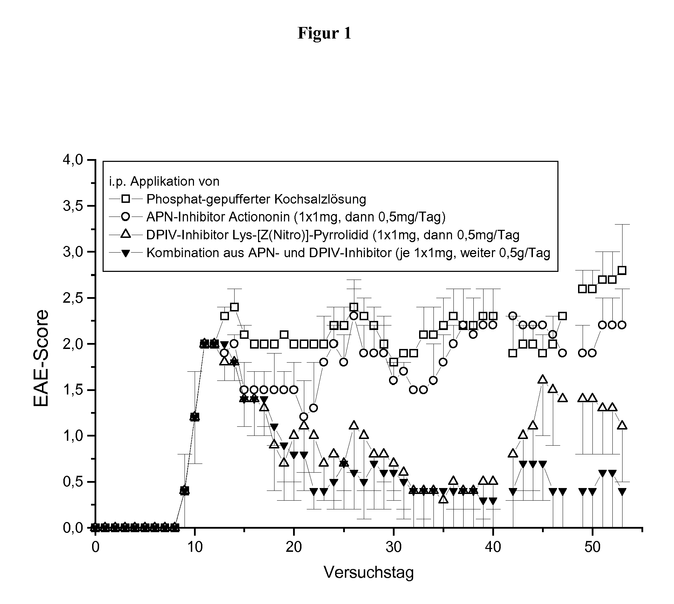

Die Erkrankung EAE wurde durch tägliche Injektion von SJL/J-Mäusen (n = 10) mit PLP139-151 (myelin antigen proteolipid protein peptide 139-151) induziert. Nach Ausbruch der Erkrankung erfolgte am 11.Tag nach Immunisierung eine therapeutische Intervention durch intraperitoneale Injektion von jeweils 1 mg der Peptidase-Inhibitoren am ersten Tag und weiteren Injektionen von 0,5 mg der Inhibitoren jeden zweiten Tag. Die Krankheitsscores sind durch unterschiedlich stark ausgeprägte Lähmungsgrade definiert. Gesunde Tier haben den Krankheitsscore 0. Als Alanyl-Aminopeptidase-Inhibior wurde Actinonin, als Dipeptidylpeptidase-IV-Inhibitor Lys[Z(NO2)]-Pyrrolidid verwendet Die Behandlung erfolgte über 46 Tage nach Immunisierung. Die Ergebnisse sind aus Figur 1 ersichtlich. Die Kurvenverläufe belegen eindeutig einen besonders starken und anhaltenden therapeutischen Effekt nach kombinierter Hemmung beider Peptidasen.

### Beispiel 3:

### Therapeutische Wirkung der kombinierten Hemmung der Alanyl-Aminopeptidasen und analog wirkender Enzyme sowie der Dipeptidylpeptidase IV und analog wirkender Enzyme auf die Dextranulfat-induzierte Colitis der Maus als Tiermodell für chronisch entzündliche Darmerkrankungen.

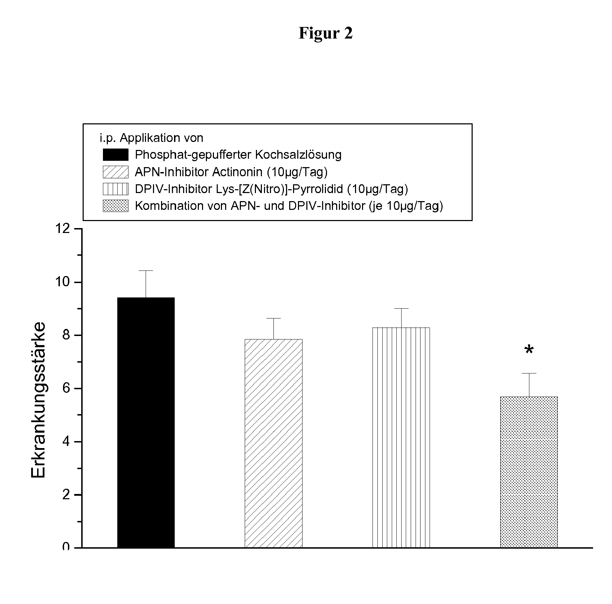

Eine vorwiegend das Colon betreffende Entzündung (äquivalent zum Krankheitsbild der Colitis ulcerosa am Menschen) wurde durch Verabreichung von 3% Natriumdextransulfat im Trinkwasser bei 8 Wochen alten, weiblichen Balb/c-Mäusen induziert. Nach 3 Tagen zeigen alle Tiere eine deutliche, erkrankungstypische Symptomatik. Die Peptidase-Inhibitoren bzw. die Phosphat-gepufferte Kochsalzlösung als Placebo wurden intraperitoneal ab Tag 5 an drei aufeinanderfolgenden Tage verabreicht. Der Schweregrad wird anhand eines anerkannten Bewertungssystems (Score) ermittelt. Dabei fließen folgende Parameter in die Bewertung ein: Stuhlkonsistenz (fest = 0 Punkte (Pkt.), pastös = 2 Pkt., flüssig/durchfallartig = 4 Pkt); Blutnachweis im Kot (negativ = 0 Pkt., okkult = 2 Pkt., deutlich sichtbar = 4 Pkt.); Gewichtsverlust (0-5% = 0 Pkt., 5-10% = 1 Pkt., 10-15% = 2 Pkt., 15-20% = 3 Pkt., <20% = 4 Pkt.). Gesunde Tiere haben einen Score-Wert von 0 Punkten, maximal erreichbar sind 12 Punkte. Ab einem Scorewert von 10 Punkten ist die Erkrankung potentiell tödlich. Im Erkrankungsverlauf erhöht sich der Scorewert zunächst durch Veränderung der Stuhlparameter, im späteren Verlauf (ab Tag 5) führt der Gewichtsverlust zur Steigerung des Punktewertes. Figur 2 zeigt die Erkrankungsstärke bei unbehandelten und behandelten Tieren am Versuchstag 7 nach dreitägiger Therapie.

Bei Applikation von 10µg der einzelnen Inhibitoren (N=14 pro Gruppe, siehe Legende) wurde eine leichte, jedoch nicht signifikante Verringerung des Erkrankungsschweregrades erzielt (-16,5% durch Actinonin; -12,3% durch Lys[Z(N02)]-Pyrrolidid). Bei i.p.-Applikation einer Kombination beider Peptidaseinhibitoren erfolgte eine statitisch signifikante (p=0,00189) Verbesserung des Erkrankungsschweregrades um 40%.

### Beispiel 4:

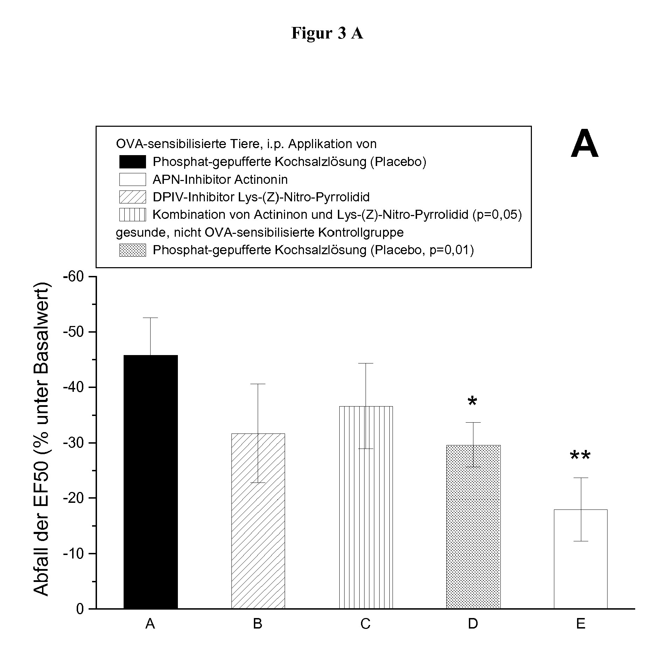

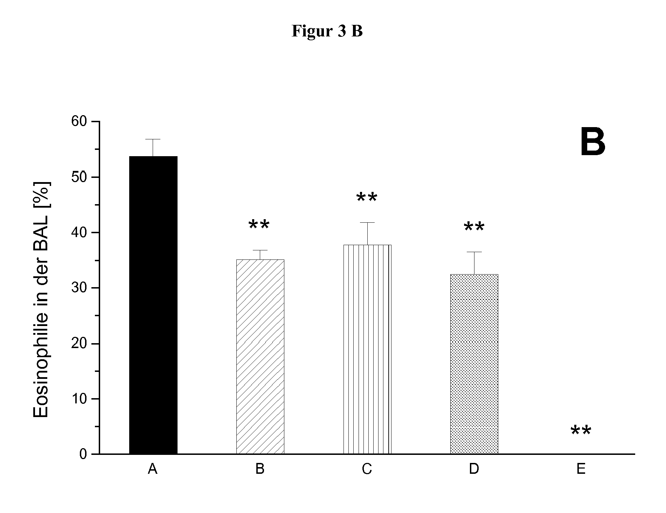

**Therapeutische Wirkung der kombinierten Hemmung der Alanyl-Aminopeptidasen und analog wirkender Enzyme sowie der Dipeptidylpeptidase IV und analog wirkender Enzyme auf das Ovalbumin-induzierten Asthma bronchiale der Maus als Tiermodell für das humanen Asthma bronchiale. Dargestellt ist der Einfluß der kombinierten Peptidase-Hemmung auf den Abfalls des mittelexpiratorischen Flusses EF 50 als Maß der Lungenfunktion (****Figur 3 A)** **sowie auf die Eosinophilie als Charakteristikum der Entzündung der Lunge bei Asthma bronchiale (****Figur 3 B)**.

Die Sensibilisierung für das Asthma bronchiale induzierende Antigen Ovalbumin erfolgte an weiblichen Balb/c-Mäusen durch intraperitoneale Gabe von je 10 µg Ovalbumin an den Tagen 0, 14 und 21. Am Tag 27/28 wurden die Tiere mit Ovalbumin inhalativ geboostert. Nach intraperitonealer Applikation der Peptidase-Inhibitoren an den Tagen 28-35 erfolgte am Tag 35 eine intranasale Ovalbumin-Challenge und eine Überprüfung der allergischen Frühreaktion über die Lungenfuktion. Gemessen wurden der mittelexpiratorische Fluß EF50, das Atemzugvolumen, die Atemfrequenz und das Minutenvolumen sowie die Zahl der eosinophilen Granulozyten in der bronchoalveolären Lavage. Pro Versuchsgruppe wurden 8-10 Tiere eingesetzt. In Figur 3A sind beispielhaft die Wirkungen der Peptidase-Inhibitoren auf die Reduktion des Abfalls von EF50 zusammengestellt. Sowohl der Alanyl-Aminopeptidase-Inhibitor Actinonin (Gruppe B; 0.1 mg) als auch der Dipeptidylpeptidase-Inhibitor Lys[Z(NO2)]-pyrrolidid (Gruppe C; 0.1 mg) zeigten therapeutische Wirkungen. Signifikante therapeutische Effekte wurden allerdings nur mit Kombinationen beider Inhibitoren (Gruppe D; je 0,1 mg) erzielt.

Gruppe E repräsentiert Tiere, die nicht OVA-sensibilisiert wurden, jedoch ansonsten allen Proceduren unterzogen wurden, die die Tiere der Gruppen A bis D durchlaufen haben. Es handelt sich bei dieser Gruppe somit um gesunde, nicht-allergische Tiere, die es aber ermöglichen Stress induzierte Effekte auf die Lungenfunktion kalkulieren zu können.

Die vorliegende Erfindung betrifft Verbindungen der allgemeinen Formel C1 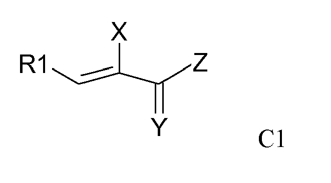 worin
- X und Z gleich oder verschieden sein können und unabhängig voneinander gewählt sind aus der Gruppe, die besteht aus Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl und Amino (NH2, NHR2, NR2R3);
- Y für O, S oder NR4 steht;
- R1, R2, R3 und R4 gleich oder verschieden sein können und gewählt sind aus der Gruppe, die besteht aus Wasserstoff, unsubstituiertem oder substituiertem, geradkettigem oder verzweigtem C₁- bis C₁₂- Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio unsubstituiertem oder substituiertem, unkondensiertem oder kondensiertem, gegebenenfalls ein oder mehrere Heteroatome aus der Gruppe N, O, P und S enthaltendem Aryl und Cycloalk-yl, unsubstituiertem oder substituiertem Amino, unsubstituiertem oder substituiertem Carbonyl, unsubstituiertem oder substituiertem Thiocarbonyl und unsubstituiertem oder substituiertem Imino; und
- die heteroaromatischen oder heterocyclischen Reste über ein C-Atom oder ein Heteroatom mit der Grundstruktur der allgemeinen Formel C1 verbunden sind;
- und Tautomere, Stereoisomere der Verbindungen der allgemeinen Formel C1 und pharmazeutisch akzeptable Salze, Salzderivate, Tautomere und Stereoisomere davon, für die Verwendung in der Medizin.

In einer bevorzugten Ausführungsform betrifft die vorliegende Erfindung Verbindungen der allgemeinen Formel C1 für die Verwendung in der Medizin, nämlich Verbindungen, die beispielsweise, aber nicht ausschliesslich gewählt sind aus der folgenden Gruppe C1 nach Table 1, und Tautomere, Stereoisomere der Verbindungen und pharmazeutisch akzeptable Salze, Salzderivate, Tautomere und Stereoisomere davon:

**Table 1:**

| **Compound ID.** | **Structure** |
|---|---|
| C1.001 | 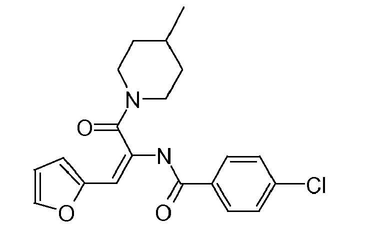 |
| C1.002 | 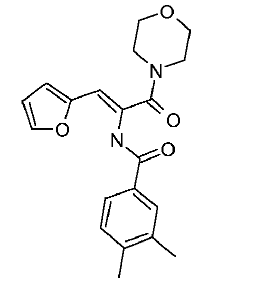 |
| C1.003 | 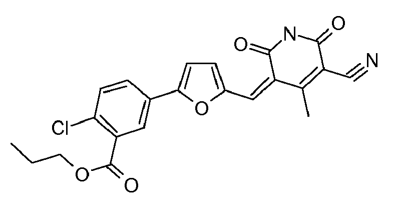 |

Alternativ dazu betrifft die vorliegende Erfindung Verbindungen der allgemeinen Formel C2 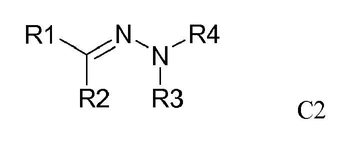 worin
- R1 bis R4 gleich oder verschieden sein können und gewählt sind aus der Gruppe, die besteht aus Wasserstoff, unsubstituiertem oder substituiertem, geradkettigem oder verzweigtem C₁- bis C₁₂- Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carbonyl, unsubstituiertem oder substituiertem Thiocarbonyl und unsubstituiertem oder substituiertem Imino; und
- die heteroaromatischen oder heterocyclischen Reste über ein C-Atom oder ein Heteroatom mit der Grundstruktur der allgemeinen Formel C2 verbunden sind;
- und Tautomere, Stereoisomere der Verbindungen der allgemeinen Formel C2 und pharmazeutisch akzeptable Salze, Salzderivate, Tautomere und Stereoisomere davon, für die Verwendung in der Medizin.

In einer bevorzugten Ausführungsform betrifft die vorliegende Erfindung Verbindungen der allgemeinen Formel C2 für die Verwendung in der Medizin, nämlich Verbindungen, die beispielsweise, aber nicht ausschliesslich gewählt sind aus der folgenden Gruppe C2 nach Table 2, und Tautomere, Stereoisomere der Verbindungen und pharmazeutisch akzeptable Salze, Salzderivate, Tautomere und Stereoisomere davon:

**Table 2:**

| **Compound ID.** | **Structure** |
|---|---|
| C2.001 | 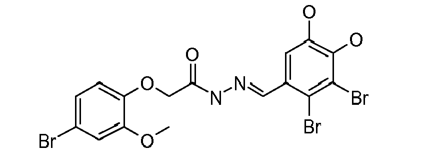 |
| C2.002 | 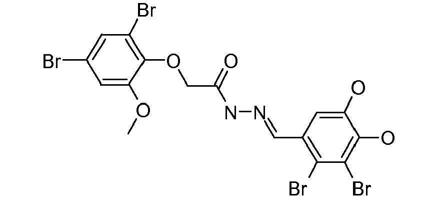 |
| C2.003 | 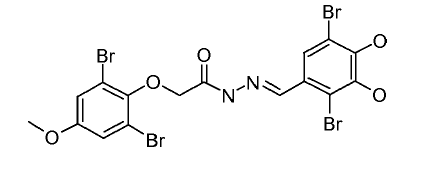 |
| C2.004 | 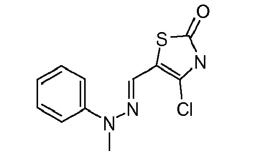 |
| C2.005 | 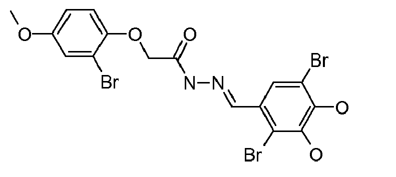 |
| C2.006 | 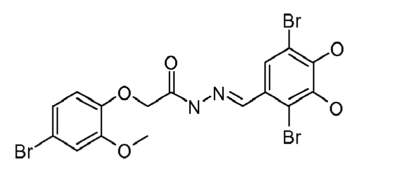 |
| C2.007 | 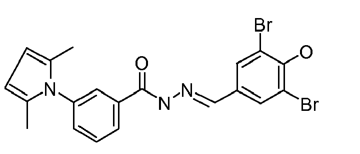 |
| C2.008 | 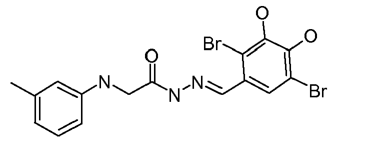 |
| C2.009 | 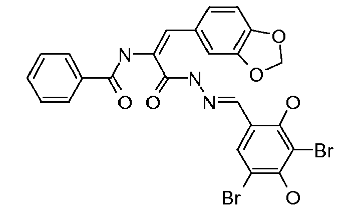 |
| C2.010 | 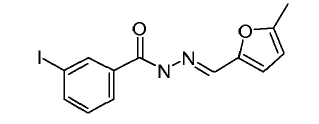 |
| C2.011 | 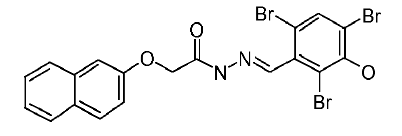 |
| C2.012 | 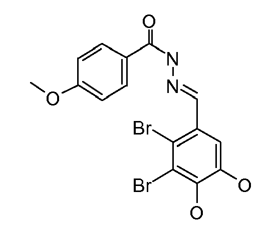 |
| C2.013 | 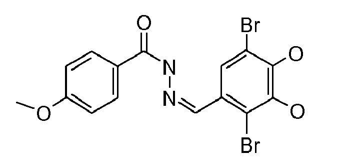 |
| C2.014 | 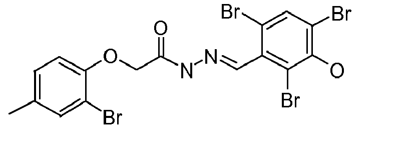 |
| C2.015 | 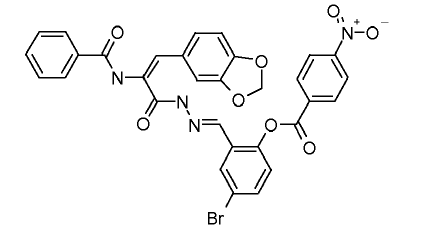 |

In einer weiteren Alternative betrifft die vorliegende Erfindung Verbindungen der allgemeinen Formel C3 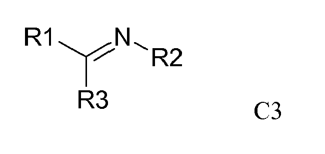 worin
- R1, R2 und R3 gleich oder verschieden sein können und gewählt sind aus der Gruppe, die besteht aus Wasserstoff, unsubstituiertem oder substituiertem, geradkettigem oder verzweigtem C₁- bis C₁₂-Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carbonyl, unsubstituiertem oder substituiertem Thiocarbonyl und unsubstituiertem oder substituiertem Imino; und
- die heteroaromatischen oder heterocyclischen Reste über ein C-Atom oder ein Heteroatom mit der Grundstruktur der allgemeinen Formel C3 verbunden sind;
- und Tautomere, Stereoisomere der Verbindungen der allgemeinen Formel C3 und pharmazeutisch akzeptable Salze, Salzderivate, Tautomere und Stereoisomere davon, für die Verwendung in der Medizin.

In einer bevorzugten Ausführungsform betrifft die vorliegende Erfindung Verbindungen der allgemeinen Formel C3 für die Verwendung in der Medizin, nämlich Verbindungen, die beispielsweise, aber nicht ausschliesslich gewählt sind aus der folgenden Gruppe C3 nach Table 3, und Tautomere, Stereoisomere der Verbindungen und pharmazeutisch akzeptable Salze, Salzderivate, Tautomere und Stereoisomere davon:

**Table 3:**

| **Compound ID.** | **Structure** |
|---|---|
| C3.001 | 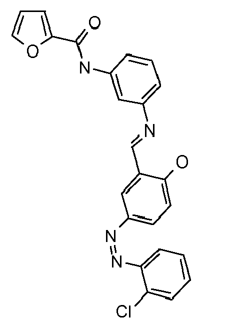 |
| C3.002 | 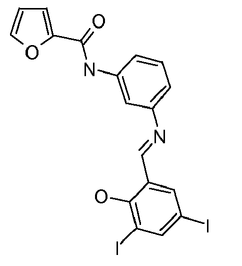 |
| C3.004 | 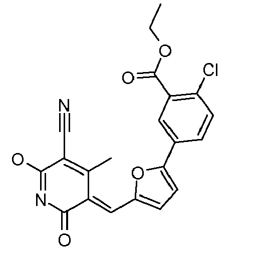 |

In einer weiteren Alternative betrifft die vorliegende Erfindung Verbindungen der allgemeinen Formel C4 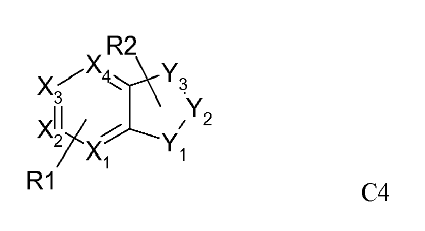 worin
- X1, X2, X3 und X4 gleich oder verschieden sein können und CH oder CR3-Einheiten darstellen;
- Y1, Y2 und Y3 gleich oder verschieden sein können substituierte oder nichtsubstituierte Kohlenstoff- oder Heteroatomeinheiten mit den Ringatomen N, O, P oder S darstellen;
- R1 und R2 das Substitutionsmuster des jeweiligen Teilringes symbolisieren, wobei R1 für einen bis vier und R2 für einen bis sechs gleiche oder verschiedene Substituenten stehen, und gewählt sind aus der Gruppe, die besteht aus Wasserstoff, unsubstituiertem oder substituiertem, geradkettigem oder verzweigtem C₁- bis C₁₂-Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carbonyl, unsubstituiertem oder substituiertem Thiocarbonyl und unsubstituiertem oder substituiertem Imino; und
- die heteroaromatischen oder heterocyclischen Reste über ein C-Atom oder ein Heteroatom mit der Grundstruktur der allgemeinen Formel C4 verbunden sind;
- und Tautomere, Stereoisomere der Verbindungen der allgemeinen Formel C4 und pharmazeutisch akzeptable Salze, Salzderivate, Tautomere und Stereoisomere davon, für die Verwendung in der Medizin.

In einer bevorzugten Ausführungsform betrifft die vorliegende Erfindung Verbindungen der allgemeinen Formel C4 für die Verwendung in der Medizin, nämlich Verbindungen, die beispielsweise, aber nicht ausschliesslich gewählt sind aus der folgenden Gruppe C4 nach Table 4, und Tautomere, Stereoisomere der Verbindungen und pharmazeutisch akzeptable Salze, Salzderivate, Tautomere und Stereoisomere davon:

**Table 4:**

| **Compound ID.** | **Structure** |
|---|---|
| C4.002 | 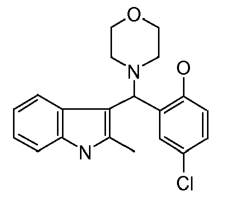 |
| C4.005 | 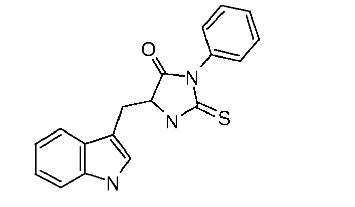 |
| C4.006 | 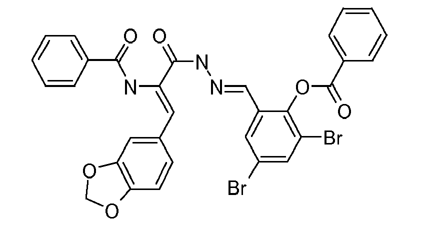 |
| C4.007 | 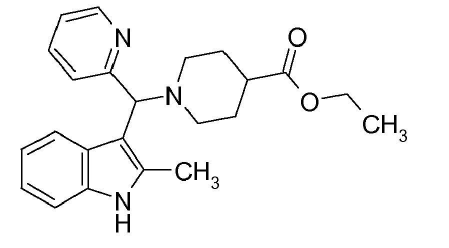 |
| C4.008 | 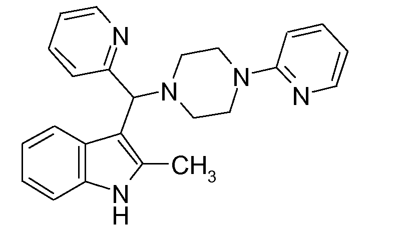 |
| C4.009 | 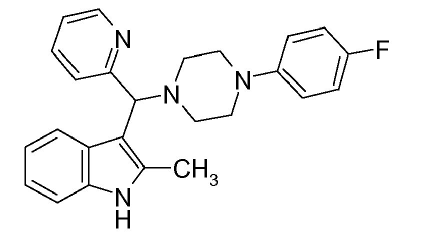 |
| C4.010 | 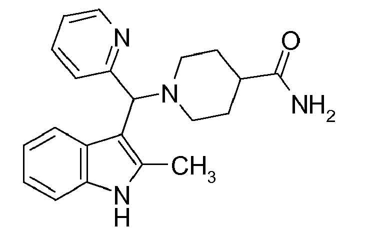 |
| C4.011 | 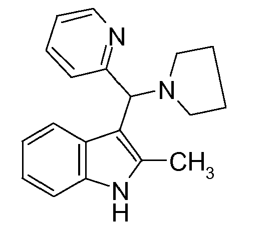 |
| C4.012 | 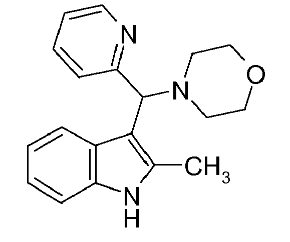 |
| C4.013 | 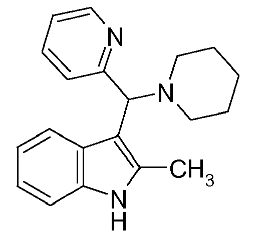 |
| C4.014 | 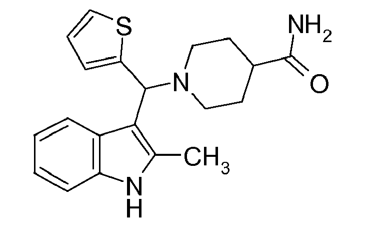 |
| C4.015 | 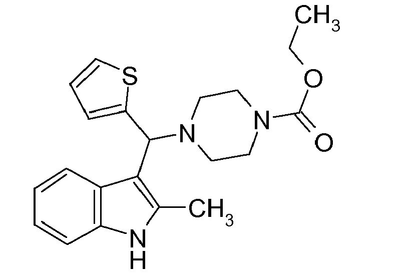 |
| C4.016 | 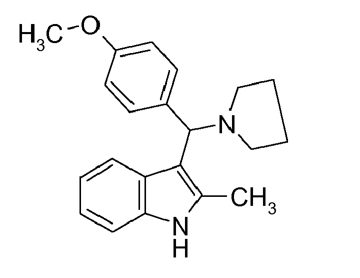 |
| C4.017 | 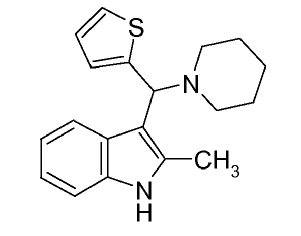 |
| C4.018 | 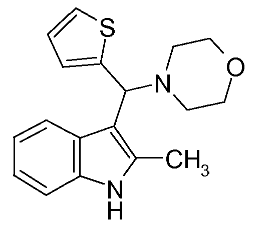 |
| C4.019 | 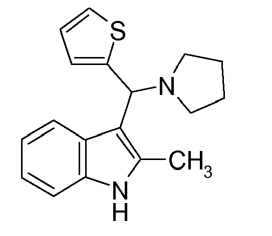 |

In einer weiteren Alternative betrifft die vorliegende Erfindung Verbindungen der allgemeinen Formel C5 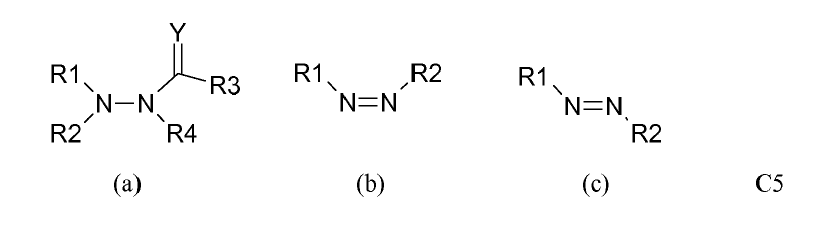 worin
- Y für O, S, NH oder NR5 stehen kann;
- R1 bis R5 gleich oder verschieden sein können und gewählt sind aus der Gruppe, die besteht aus Wasserstoff, unsubstituiertem oder substituiertem, geradkettigem oder verzweigtem C₁- bis C₁₂-Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carbonyl, unsubstituiertem oder substituiertem Thiocarbonyl und unsubstituiertem oder substituiertem Imino; und
- die heteroaromatischen oder heterocyclischen Reste über ein C-Atom oder ein Heteroatom mit der Grundstruktur der allgemeinen Formel C5 verbunden sind;
- und Tautomere, Stereoisomere der Verbindungen der allgemeinen Formel C5 und pharmazeutisch akzeptable Salze, Salzderivate, Tautomere und Stereoisomere davon, für die Verwendung in der Medizin.

In einer bevorzugten Ausführungsform betrifft die vorliegende Erfindung Verbindungen der allgemeinen Formel C5 für die Verwendung in der Medizin, nämlich Verbindungen, die beispielsweise aber nicht ausschliesslich gewählt sind aus der folgenden Gruppe C5 nach Table 5, und Tautomere, Stereoisomere der Verbindungen und pharmazeutisch akzeptable Salze, Salzderivate, Tautomere und Stereoisomere davon:

**Table 5:**

| **Compound ID.** | **Structure** |
|---|---|
| C5.001 | 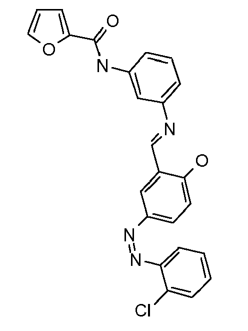 |
| C5.002 | 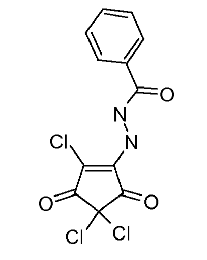 |

In einer weiteren Alternative betrifft die vorliegende Erfindung Verbindungen der allgemeinen Formel C6 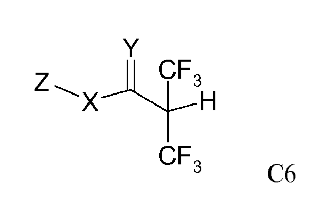 worin
- Y für O, S, NH oder NR1 steht;
- X und Z gleich oder verschieden sein können und unabhängig voneinander gewählt sind aus der Gruppe, die besteht aus Hydroxy, Thiol, C₁- bis C₈-Alkoxy, C₁- bis C₈-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl und Amino (NH2, NHR2, NR2R3);
- R1 bis R3 gleich oder verschieden sein können und gewählt sind aus der Gruppe, die besteht aus Wasserstoff, unsubstituiertem oder substituiertem, geradkettigem oder verzweigtem C₁- bis C₁₂- Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carbonyl, unsubstituiertem oder substituiertem Thiocarbonyl und unsubstituiertem oder substituiertem Imino; und
- die heteroaromatischen oder heterocyclischen Reste über ein C-Atom oder ein Heteroatom mit der Grundstruktur der allgemeinen Formel C6 verbunden sind;
- und Tautomere, Stereoisomere der Verbindungen der allgemeinen Formel C6 und pharmazeutisch akzeptable Salze, Salzderivate, Tautomere und Stereoisomere davon, für die Verwendung in der Medizin.
   In einer bevorzugten Ausführungsform betrifft die vorliegende Erfindung Verbindungen der allgemeinen Formel C6 für die Verwendung in der Medizin, nämlich Verbindungen, die beispielsweise aber nicht ausschliesslich gewählt sind aus der folgenden Gruppe C6 nach Table 6, und Tautomere, Stereoisomere der Verbindungen und pharmazeutisch akzeptable Salze, Salzderivate, Tautomere und Stereoisomere davon:

**Table 6:**

| **Compound ID.** | **Structure** |
|---|---|
| C6.001 | 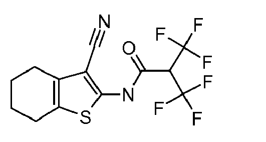 |
| C6.002 | 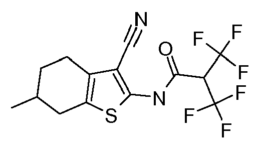 |

In einer weiteren Alternative betrifft die vorliegende Erfindung Verbindungen der allgemeinen Formel C7 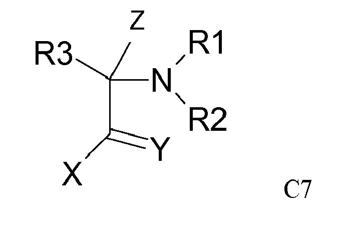 worin
- Y für O, S, NH oder NR4 steht;
- X und Z gleich oder verschieden sein können und unabhängig voneinander gewählt sind aus der Gruppe, die besteht aus Hydroxy, Thiol, C₁- bis C₈-Alkoxy, C₁- bis C₈-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl und Amino (NH2, NHR5, NR5R6);
- R1 bis R6 gleich oder verschieden sein können und gewählt sind aus der Gruppe, die besteht aus Wasserstoff, unsubstituiertem oder substituiertem, geradkettigem oder verzweigtem C₁- bis C₁₂-Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carbonyl, unsubstituiertem oder substituiertem Thiocarbonyl und unsubstituiertem oder substituiertem Imino; und
- die heteroaromatischen oder heterocyclischen Reste über ein C-Atom oder ein Heteroatom mit der Grundstruktur der allgemeinen Formel C7 verbunden sind;
- und Tautomere, Stereoisomere der Verbindungen der allgemeinen Formel C7 und pharmazeutisch akzeptable Salze, Salzderivate, Tautomere und Stereoisomere davon, für die Verwendung in der Medizin.
   In einer bevorzugten Ausführungform betrifft die vorliegende Erfindung Verbindungen der allgemeinen Formel C7 für die Verwendung in der Medizin, nämlich Verbindungen, die beispielsweise, aber nicht ausschliesslich gewählt sind aus der folgenden Gruppe C7 nach Table 7, und Tautomere, Stereoisomere der Verbindungen und pharmazeutisch akzeptable Salze, Salzderivate, Tautomere und Stereoisomere davon:

**Table 7:**

| **Compound ID.** | **Structure** |
|---|---|
| C7.001 | 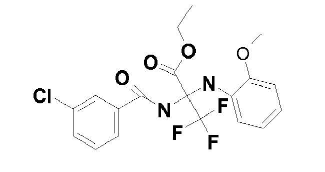 |
| C7.002 | 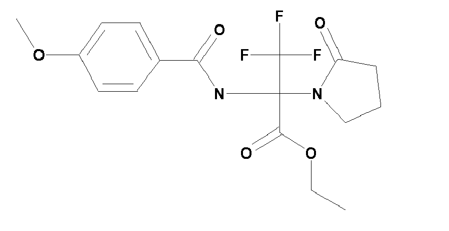 |
| C7.003 | 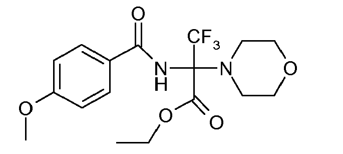 |
| C7.004 | 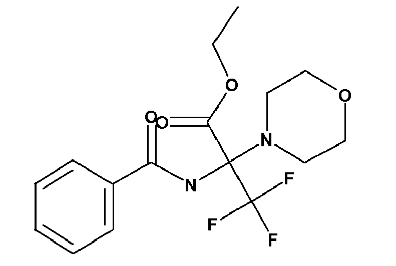 |

In einer weiteren Alternative betrifft die vorliegende Erfindung Verbindungen der allgemeinen Formel C8 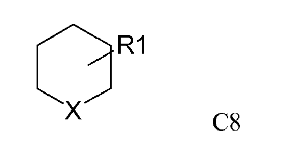 worin
- X für ein Heteroatom N, O, S oder P steht oder eine eines dieser Heteroatome als Ringatom enthaltende funktionelle Gruppe darstellt;
- die Sechsring-Grundstruktur bis zu drei weitere Heteroatome der Gruppe X enthalten kann, wobei die Heteroatome gleich oder verschieden sein können;
- die Sechsring-Grundstruktur null bis drei Doppelbindungen enthalten kann;
- R1 die Substitution der Sechsring-Grundstruktur symbolisiert und für einen bis zehn Substituenten steht;
- R1 gewählt ist aus der Gruppe, die besteht aus Wasserstoff, unsubstituiertem oder substituiertem, geradkettigem oder verzweigtem C₁- bis C₁₂-Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carbonyl, unsubstituiertem oder substituiertem Thiocarbonyl und unsubstituiertem oder substituiertem Imino; und
- die heteroaromatischen oder heterocyclischen Reste über ein C-Atom oder ein Heteroatom mit der Grundstruktur der allgemeinen Formel C8 verbunden sind;
- und Tautomere, Stereoisomere der Verbindungen der allgemeinen Formel C8 und pharmazeutisch akzeptable Salze, Salzderivate, Tautomere und Stereoisomere davon, für die Verwendung in der Medizin.

In einer bevorzugten Ausführungsform betrifft die vorliegende Erfindung Verbindungen der allgemeinen Formel C8 für die Verwendung in der Medizin, nämlich Verbindungen, die beispielsweise, aber nicht ausschliesslich gewählt sind aus der folgenden Gruppe C8 nach Table 8, und Tautomere, Stereoisomere der Verbindungen und pharmazeutisch akzeptable Salze, Salzderivate und Tautomere und Stereoisomere davon:

**Table 8:**

| **Compound ID** | **Structure** |
|---|---|
| C8.001 | 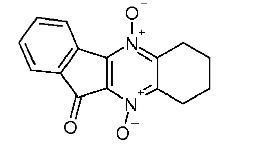 |
| C8.002 | 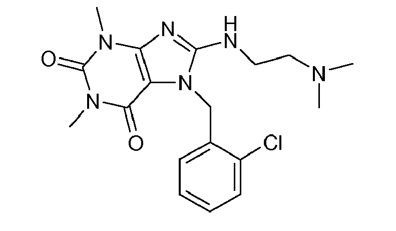 |
| C8.003 | 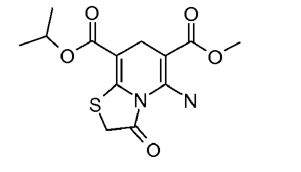 |
| C8.004 | 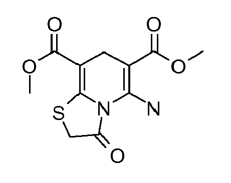 |
| C8.005 | 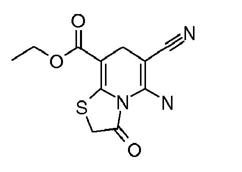 |
| C8.006 | 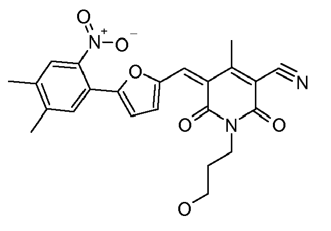 |
| C8.007 | 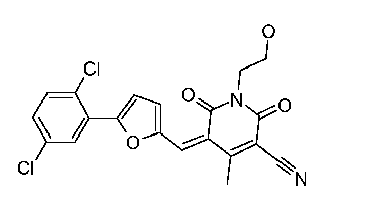 |
| C8.008 | 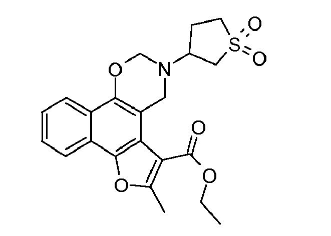 |
| C8.009 | 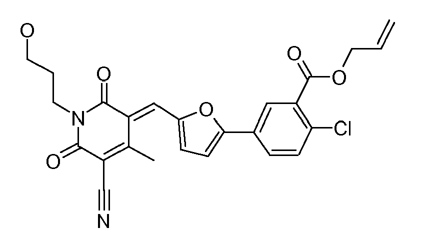 |
| C8.010 | 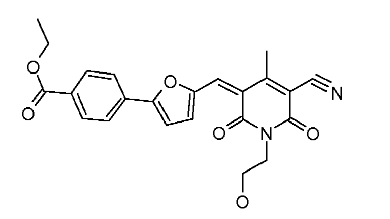 |
| C8.011 | 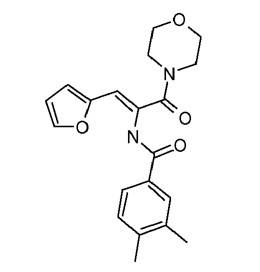 |
| C8.012 | 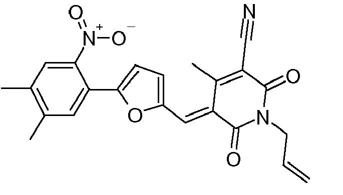 |
| C8.013 | 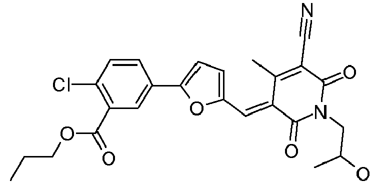 |
| C8.014 | 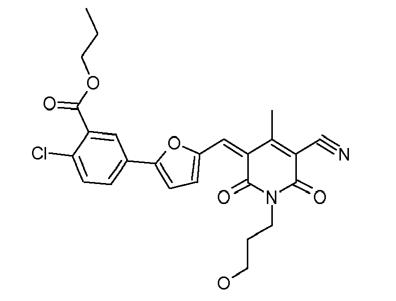 |
| C8.015 | 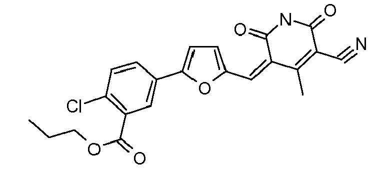 |
| C8.016 | 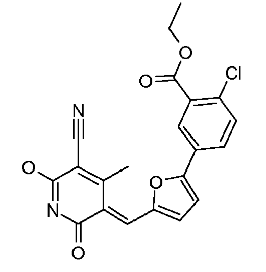 |
| C8.017 | 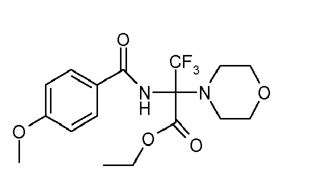 |
| C8.018 | 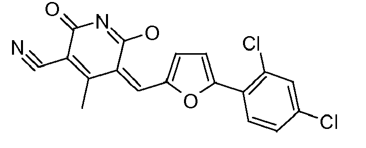 |
| C8.019 | 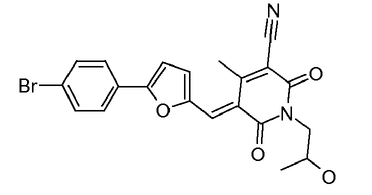 |
| C8.020 | 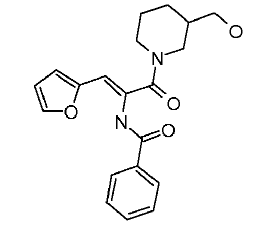 |
| C8.021 | 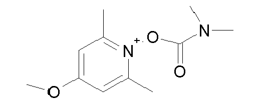 |
| C8.022 | 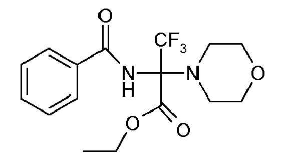 |
| C8.023 | 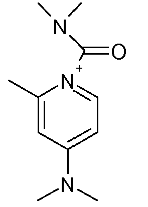 |

In einer weiteren Alternative betrifft die vorliegende Erfindung Verbindungen der allgemeinen Formel C9 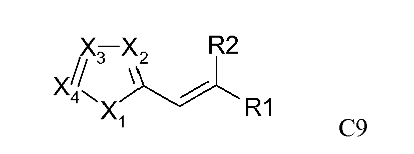 worin
- X1 für CH₂, CHR3, CR3R4, NH, NR4, O oder S stehen kann;
- X2, X3 und X4 für CH, CR5 oder N stehen können;
- R1 bis R5 gleich oder verschieden sein können und gewählt sind aus der Gruppe, die besteht aus Wasserstoff, unsubstituiertem oder substituiertem, geradkettigem oder verzweigtem C₁- bis C₁₂-Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carbonyl, unsubstituiertem oder substituiertem Thiocarbonyl und unsubstituiertem oder substituiertem Imino;
- die heteroaromatischen oder heterocyclischen Reste über ein C-Atom oder ein Heteroatom mit der Grundstruktur der allgemeinen Formel C9 verbunden sind;
- und die Sechsring-Grundstruktur null bis drei Doppelbindungen enthalten kann
- und Tautomere, Stereoisomere der Verbindungen der allgemeinen Formel C9 und pharmazeutisch akzeptable Salze, Salzderivate, Tautomere und Stereoisomere davon, für die Verwendung in der Medizin.

In einer bevorzugten Ausführungsform betrifft die vorliegende Erfindung Verbindungen der allgemeinen Formel C9 für die Verwendung in der Medizin, nämlich Verbindungen, die beispielsweise, aber nicht ausschliesslich gewählt sind aus der folgenden Gruppe C9 nach Table 9, und Tautomere, Stereoisomere der Verbindungen und pharmazeutisch akzeptable Salze, Salzderivate, Tautomere und Stereoisomere davon:

**Table 9:**

| **Compound ID.** | **Structure** |
|---|---|
| C9.001 | 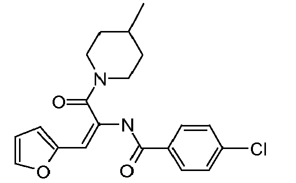 |
| C9.002 | 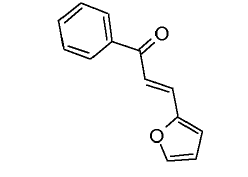 |
| C9.003 | 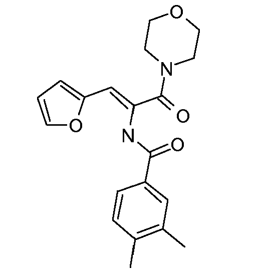 |
| C9.004 | 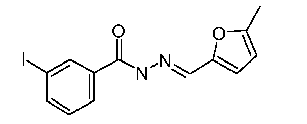 |
| C9.005 | 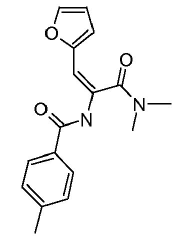 |
| C9.006 | 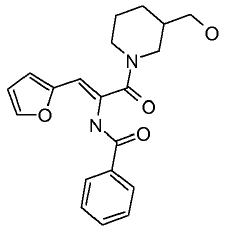 |

In einer weiteren Alternative betrifft die vorliegende Erfindung Verbindungen der allgemeinen Formel C10 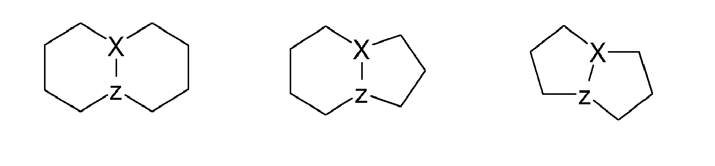 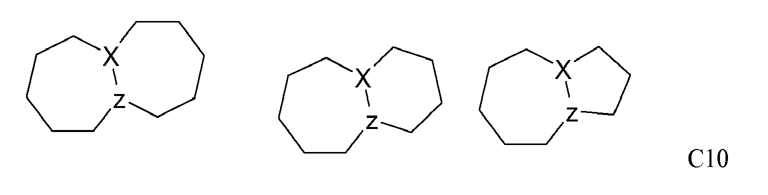 worin
- X und Z für CH, CR1 oder N stehen können und mindestens eine der beiden Gruppen ein Heteroatom der Grundstruktur darstellt oder besitzt;
- die Teilringe substituiert oder nichtsubstituiert, kondensiert oder nichtkondensiert sein können und null bis drei Doppelbindungen und weitere Heteroatome und Heteroatome enthaltenden Gruppen entsprechend der Definitionen für X und Z enthalten können,
- R1 gewählt ist aus der Gruppe, die besteht aus Wasserstoff, unsubstituiertem oder substituiertem, geradkettigem oder verzweigtem C₁- bis C₁₂- Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carbonyl, unsubstituiertem oder substituiertem Thiocarbonyl und unsubstituiertem oder substituiertem Imino;
- die heteroaromatischen oder heterocyclischen Reste über ein C-Atom oder ein Heteroatom mit der Grundstruktur der allgemeinen Formel C10 verbunden sind;
- die Ringsysteme der Grundstrukturen null bis drei Doppelbindungen enthalten kann und
- und Tautomere, Stereoisomere der Verbindungen der allgemeinen Formel C10 und pharmazeutisch akzeptable Salze, Salzderivate, Tautomere und Stereoisomere davon, für die Verwendung in der Medizin.

In einer bevorzugten Ausführungsform betrifft die vorliegende Erfindung Verbindungen der allgemeinen Formel C10 für die Verwendung in der Medizin, nämlich Verbindungen, die beispielsweise, aber nicht ausschliesslich gewählt sind aus der folgenden Gruppe C10 nach Table 10, und Tautomere, Stereoisomere der Verbindungen und pharmazeutisch akzeptable Salze, Salzderivate, Tautomere und Stereoisomere davon:

**Table 10:**

| **Compound ID.** | **Structure** |
|---|---|
| C10.003 | 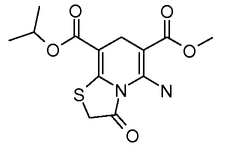 |
| C10.005 | 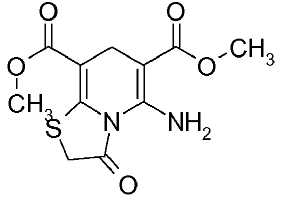 |
| C10.012 | 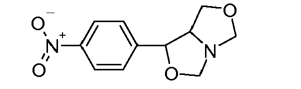 |
| C10.015 | 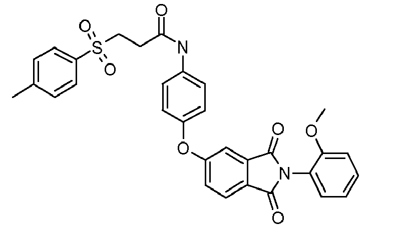 |

In einer weiteren Alternative betrifft die vorliegende Erfindung Verbindungen der allgemeinen Formel C11 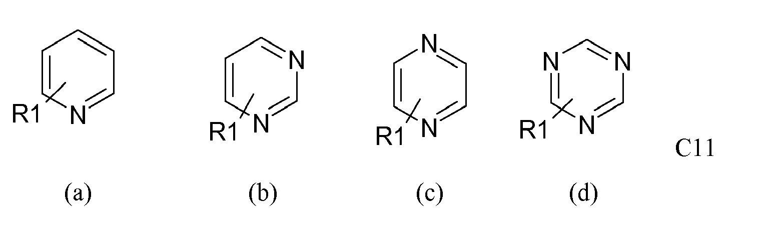 worin
- R1 für das aus bis zu fünf gleichen oder verschiedenen Substituenten bestehende Substitutionsmuster der heteroaromatischen Grundstruktur steht und gewählt ist aus der Gruppe, die besteht aus Wasserstoff, unsubstituiertem oder substituiertem, geradkettigem oder verzweigtem C₁- bis C₁₂-Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carbonyl, unsubstituiertem oder substituiertem Thiocarbonyl und unsubstituiertem oder substituiertem Imino; und
- die heteroaromatischen oder heterocyclischen Reste über ein C-Atom oder ein Heteroatom mit der Grundstruktur der allgemeinen Formel C11 verbunden sind;
- und Tautomere, Stereoisomere der Verbindungen der allgemeinen Formel C11 und pharmazeutisch akzeptable Salze, Salzderivate, Tautomere und Stereoisomere davon, für die Verwendung in der Medizin.
   In einer bevorzugten Ausführungsform betrifft die vorliegende Erfindung Verbindungen der allgemeinen Formel C11 für die Verwendung in der Medizin, nämlich Verbindungen, die beispielsweise, aber nicht ausschließlich gewählt sind aus der folgenden Gruppe C11 nach Table 11, und Tautomere, Stereoisomere der Verbindungen und pharmazeutisch akzeptable Salze, Salzderivate, Tautomere und Stereoisomere davon:

**Table 11:**

| **Compound ID.** | **Structure** |
|---|---|
| C11.001 | 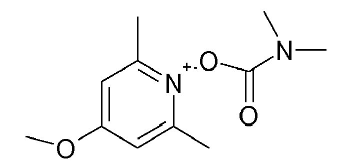 |
| C11.002 | 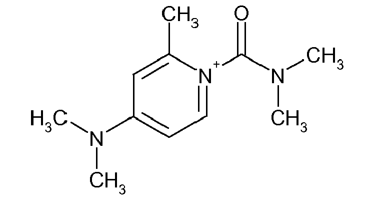 |

In einer weiteren Alternative betrifft die vorliegende Erfindung Verbindungen der allgemeinen Formel C12 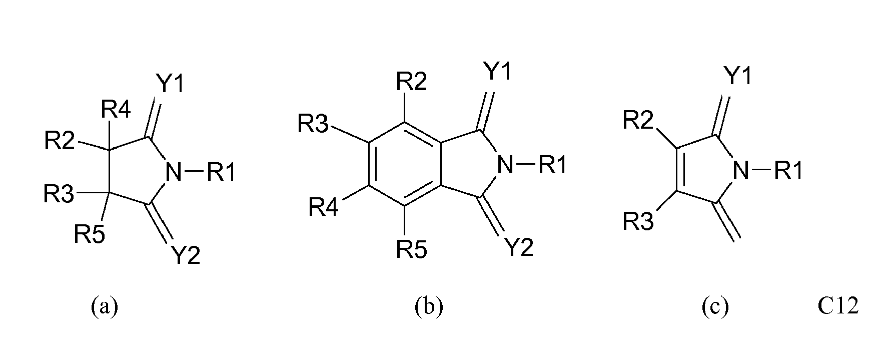 worin
- Y1 und Y2 gleich oder verschieden sein können und für O, S, NH oder NR6 stehen;
- R1 bis R6 gleich oder verschieden sein können und gewählt sind aus der Gruppe, die besteht aus Wasserstoff, unsubstituiertem oder substituiertem, geradkettigem oder verzweigtem C₁- bis C₁₂-Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubsti-tuiertem oder substituiertem Amino, unsubstituiertem oder substituiertem Carbonyl, unsubstituiertem oder substituiertem Thiocarbonyl und unsubstituiertem oder substituiertem Imino; und
- die heteroaromatischen oder heterocyclischen Reste über ein C-Atom oder ein Heteroatom mit der Grundstruktur der allgemeinen Formel C12 verbunden sind;
- und Tautomere, Stereoisomere der Verbindungen der allgemeinen Formel C12 und pharmazeutisch akzeptable Salze, Salzderivate, Tautomere und Stereoisomere davon, für die Verwendung in der Medizin.

In einer bevorzugten Ausführungsform betrifft die vorliegende Erfindung Verbindungen der allgemeinen Formel C12 für die Verwendung in der Medizin, nämlich Verbindungen, die beispielsweise, aber nicht ausschliesslich gewählt sind aus der folgenden Gruppe C12, nach Table 12, und Tautomere, Stereoisomere der Verbindungen und pharmazeutisch akzeptable Salze, Salzderivate, Tautomere und Stereoisomere davon:

**Table 12:**

| **Compound ID.** | **Structure** |
|---|---|
| C12.001 | 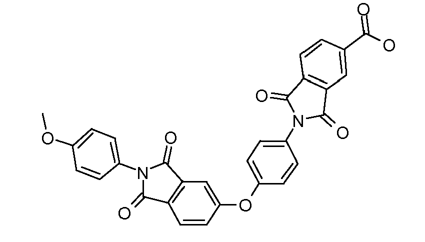 |

In einer weiteren Alternative betrifft die vorliegende Erfindung Verbindungen der allgemeinen Formel C13 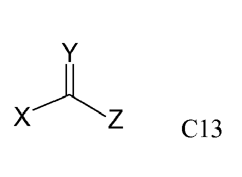 worin
- X und Z gleich oder verschieden sein können und unabhängig voneinander gewählt sind aus der Gruppe, die besteht aus Hydroxy, Thiol, C₁- bis C₈-Alkoxy, C₁- bis C₈-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl und Amino (NH₂, NH1, NR1R2);
- Y für O, S, NH oder NR3 steht;
- R1 bis R3 gleich oder verschieden sein können und gewählt sind aus der Gruppe, die besteht aus Wasserstoff, unsubstituiertem oder substituiertem, geradkettigem oder verzweigtem C₁- bis C₁₂-Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubsti-tuiertem oder substituiertem Amino, unsubstituiertem oder substituiertem Carbonyl, unsubstituiertem oder substituiertem Thiocarbonyl und unsubstituiertem oder substituiertem Imino;und
- die heteroaromatischen oder heterocyclischen Reste über ein C-Atom oder ein Heteroatom mit der Grundstruktur der allgemeinen Formel C13 verbunden sind;
- und Tautomere, Stereoisomere der Verbindungen der allgemeinen Formel C13 und pharmazeutisch akzeptable Salze, Salzderivate, Tautomere und Stereoisomere davon, für die Verwendung in der Medizin.

In einer bevorzugten Ausführungsform betrifft die vorliegende Erfindung Verbindungen der allgemeinen Formel C13 nach Anspruch 25 für die Verwendung in der Medizin, nämlich Verbindungen, die beispielsweise, aber nicht ausschliesslich gewählt sind aus der folgenden Gruppe C13 nach Table 13, und Tautomere, Stereoisomere der Verbindungen und pharmazeutisch akzeptable Salze, Salzderivate, Tautomere und Stereoisomere davon:

**Table 13:**

| **Compound ID.** | **Structure** |
|---|---|
| C13.001 | 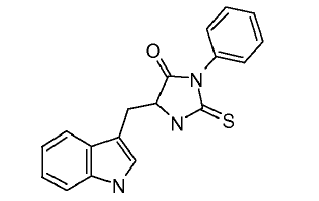 |
| C13.002 | 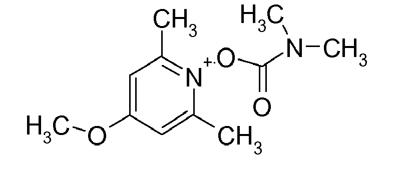 |
| C13.003 | 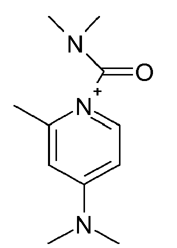 |

In einer weiteren Alternative betrifft die vorliegende Erfindung Verbindungen der allgemeinen Formel C14 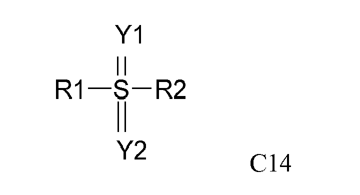 worin
- Y1 und Y2 gleich oder verschieden sein können und für O, S, NH oder NR3 stehen;
- R1 bis R3 gleich oder verschieden sein können und gewählt sind aus der Gruppe, die besteht aus Wasserstoff, unsubstituiertem oder substituiertem, geradkettigem oder verzweigtem C₁- bis C₁₂-Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carbonyl, unsubstituiertem oder substituiertem Thiocarbonyl und unsubstituiertem oder substituiertem Imino; und
- die heteroaromatischen oder heterocyclischen Reste über ein C-Atom oder ein Heteroatom mit der Grundstruktur der allgemeinen Formel C14 verbunden sind;
- und Tautomere, Stereoisomere der Verbindungen der allgemeinen Formel C14 und pharmazeutisch akzeptable Salze, Salzderivate, Tautomere und Stereoisomere davon, für die Verwendung in der Medizin.

In einer bevorzugten Ausführungsform betrifft die vorliegende Erfindung Verbindungen der allgemeinen Formel C14 für die Verwendung in der Medizin, nämlich Verbindungen, die beispielsweise, aber nicht ausschliesslich gewählt sind aus der folgenden Gruppe C14 nach Table 14, und Tautomere, Stereoisomere der Verbindungen und pharmazeutisch akzeptable Salze, Salzderivate, Tautomere und Stereoisomere davon:

**Table 14:**

| **Compound ID.** | **Structure** |
|---|---|
| C14.001 | 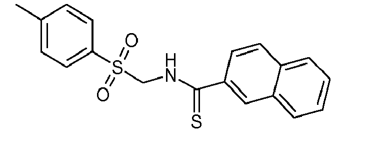 |
| C14.002 | 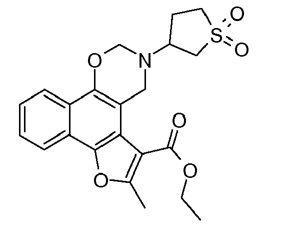 |

In einer weiteren Alternative betrifft die vorliegende Erfindung Verbindungen der allgemeinen Formel C15 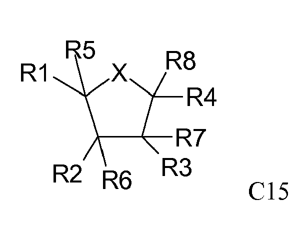 worin
- X für O, S, NH oder NR9 steht;
- R1 bis R9 gleich oder verschieden sein können und gewählt sind aus der Gruppe, die besteht aus Wasserstoff, unsubstituiertem oder substituiertem, geradkettigem oder verzweigtem C1- bis C12-Alkyl, C2- bis C12-Alkenyl und C2- bis C12-Alkinyl, Hydroxy, Thiol, C1- bis C12-Alkoxy, C1- bis C12-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carbonyl, unsubstituiertem oder substituiertem Thiocarbonyl und unsubstituiertem oder substituiertem Imino; und
- die heteroaromatischen oder heterocyclischen Reste über ein C-Atom oder ein Heteroatom mit der Grundstruktur der allgemeinen Formel C15 verbunden sind;
- und Tautomere, Stereoisomere der Verbindungen der allgemeinen Formel C15 und pharmazeutisch akzeptable Salze, Salzderivate, Tautomere und Stereoisomere davon, für die Verwendung in der Medizin.
   In einer bevorzugten Ausführungsform betrifft die vorliegenden Erfindung Verbindungen der allgemeinen Formel C15 für die Verwendung in der Medizin, nämlich Verbindungen, die beispielsweise, aber nicht ausschliesslich gewählt sind aus der folgenden Gruppe C15 nach Table 15, und Tautomere, Stereoisomere der Verbindungen und pharmazeutisch akzeptable Salze, Salzderivate, Tautomere und Stereoisomere davon:

**Table 15:**

| **Compound ID.** | **Structure** |
|---|---|
| C15.002 | 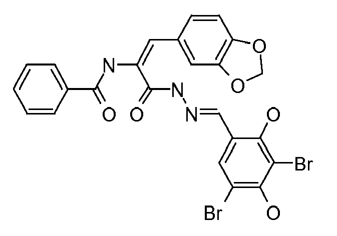 |
| C15.003 | 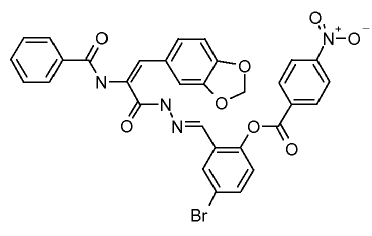 |
| C15.004 | 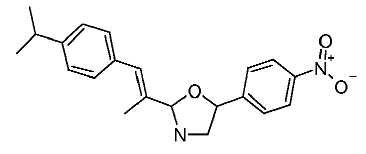 |

In einer weiteren Alternative betrifft die vorliegende Erfindung Verbindungen der allgemeinen Formel C16 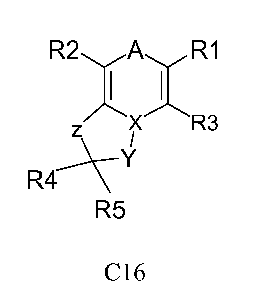 worin
- R1 bis R5 gleich oder verschieden sein können und unabhängig voneinander gewählt sind und für Wasserstoff, CH₃, CH₂R6, CHR6R7, CR6R7R8 OH, OR6, NH₂, NHR6, NR6R7, C(O)R6, C(NH)R6, C(NR7)R6, C(S)R6, PH₂, PHR6, P(R6)R7, P(O)(OH)₂, P(O)(OH)(OR6), P(O)(OR6)(OR7) und CN stehen;
- A, Y und Z gleich oder verschieden sein können und unabhängig voneinander gewählt sind und für CH₂, CHR9, CR9R10, C(O), C(S), C(NH), C(NR9), NH, NR9, NOH, NOR9, O, S, SO₂, PH, PR9, P(O)OH, P(O)OR9, P(OH)₃, P(OH)₂POR9, P(OH)(OR9)(OR10), P(OR9)(OR10)(OR11) stehen,
- X für N, CH, CR12, P, P=O, P(OH)₂, P(OH)(OR12) oder P(OR12)(OR13) steht;
- R6 bis R13 gleich oder verschieden sein können und unabhängig voneinander gewählt sind aus der Gruppe, die besteht aus Wasserstoff, unsubstituiertem oder substituiertem, geradkettigem oder verzweigtem C₁- bis C₁₂-Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carbonyl, unsubstituiertem oder substituiertem Thiocarbonyl und unsubstituiertem oder substituiertem Imino;
- die heteroaromatischen oder heterocyclischen Reste über ein C-Atom oder ein Heteroatom mit der Grundstruktur der allgemeinen Formel C16 verbunden sind;
- und Tautomere, Stereoisomere der Verbindungen der allgemeinen Formel C16 und pharmazeutisch akzeptable Salze, Salzderivate, Tautomere und Stereoisomere davon, für die Verwendung in der Medizin.

In einer weiteren Alternative betrifft die vorliegende Erfindung Verbindungen, gekennzeichnet durch folgende Grundstruktur 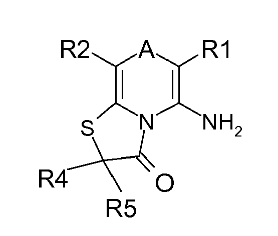 worin
- R1 bis R5 gleich oder verschieden sein können und unabhängig voneinander gewählt sind und für Wasserstoff, CH₃, CH₂R6, CHR6R7, CR6R7R8 OH, OR6, NH₂, NHR6, NR6R7, C(O)R6, C(NH)R6, C(NR7)R6, C(S)R6, PH₂, PHR6, P(R6)R7, P(O)(OH)₂, P(O)(OH)(OR6), P(O)(OR6)(OR7) und CN stehen;
- A für CH₂, CHR9, CR9R10, C(O), C(S), NH, NR9, NOH, NOR9, O, S, SO₂, PH, PR9, P(O)OH, P(O)OR9, P(OH)₃, P(OH)₂POR9, P(OH)(OR9)(OR10), P(OR9)(OR10)(OR11) steht,
- R6 bis R13 gleich oder verschieden sein können und unabhängig voneinander gewählt sind aus der Gruppe, die besteht aus Wasserstoff, unsubstituiertem oder substituiertem, geradkettigem oder verzweigtem C₁- bis C₁₂-Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carbonyl, unsubstituiertem oder substituiertem Thiocarbonyl und unsubstituiertem oder substituiertem Imino;
- und Tautomere, Stereoisomere der Verbindungen der unter 30a angegebenen allgemeinen Formel und pharmazeutisch akzeptable Salze, Salzderivate, Tautomere und Stereoisomere davon, für die Verwendung in der Medizin.

In einer bevorzugten Ausführungsform betrifft die vorliegende Erfindung für die Verwendung in der Medizin, nämlich Verbindungen, die beispielsweise, aber nicht ausschliesslich gewählt sind aus der folgenden Gruppe C16 nach Table 16, und Tautomere, Stereoisomere der Verbindungen und pharmazeutisch akzeptable Salze, Salzderivate, Tautomere und Stereoisomere davon:

**Table 16:**

| **Compound ID.** | **Structure** |
|---|---|
| C16.001 | 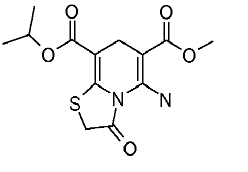 |
| C16.003 | 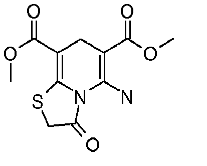 |
| C16.004 | 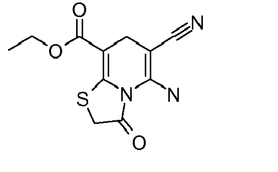 |
| C16.005 | 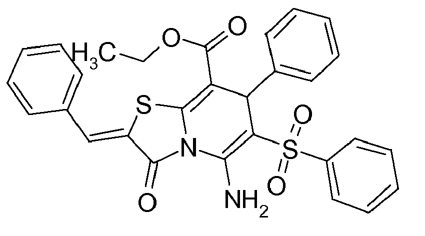 |
| C16.006 | 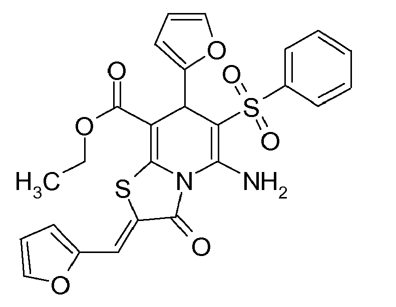 |
| C16.007 | 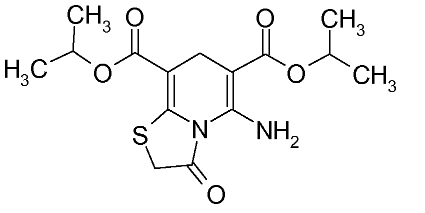 |
| C16.008 | 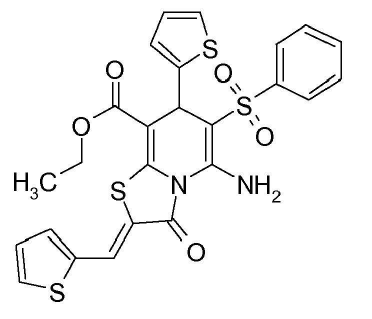 |
| C16.009 | 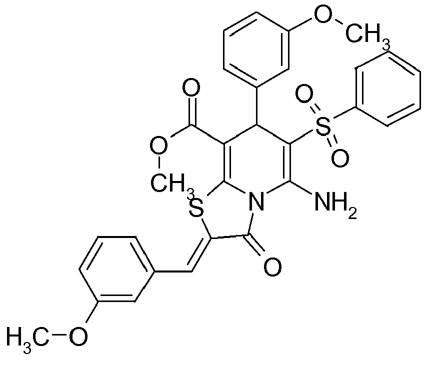 |
| C16.010 | 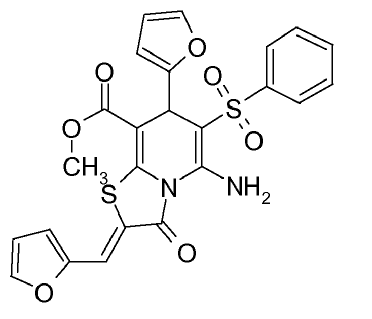 |
| C16.011 | 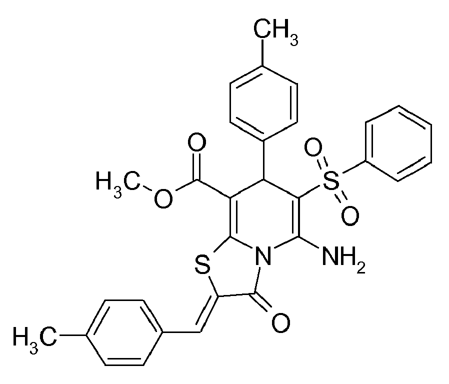 |
| C16.012 | 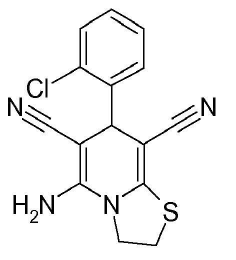 |
| C16.013 | 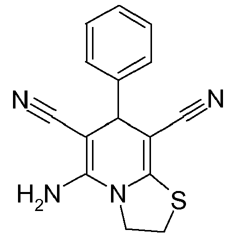 |

Darüber hinaus betrifft die vorliegende Erfindung eine pharmazeutische Zusammensetzung, welche mindestens eine Verbindung der allgemeinen Formel C1 bis C16 gegebenenfalls in Kombination mit an sich üblichen Trägern und/oder Adjuvantien umfasst.

Außerdem betrifft die vorliegende Erfindung eine kosmetische Zusammensetzung, welche mindestens eine Verbindung der allgemeinen Formel C1 bis C16 gegebenenfalls in Kombination mit an sich üblichen Trägern und/oder Adjuvantien umfasst.

Die vorliegende Erfindung betrifft auch die Verwendung von mindestens einer Verbindung der allgemeinen Formel C1 bis C16 oder die Verwendung einer pharmazeutischen oder kosmetischen Zusammensetzung, die mindestens eine Verbindung der allgemeinen Formel C 1 bis C16 umfassen, zur Hemmung der Aktivität der Alanyl-Aminopeptidasen und der Dipeptidylpeptidase oder analoger Enzyme sowie die Verwendung von mindestens einer Verbindung der allgemeinen Formel C1 bis C16 oder die Verwendung einer pharmazeutischen oder kosmetischen Zusammensetzung, die mindestens eine Verbindung der allgemeinen Formel C 1 bis C16 umfassen, zur topischen Beeinflussung der Aktivität der Alanyl-Aminopeptidasen und der Dipeptidylpeptidase oder analoger Enzyme.

In einem weiteren Aspekt betrifft die vorliegende Erfindung die Verwendung von mindestens einer Verbindung der allgemeinen Formel C1 bis C16 oder die Verwendung einer pharmazeutischen oder kosmetischen Zusammensetzung, die mindestens eine Verbindung der allgemeinen Formel C1 bis C16 umfassen,
- zur Prophylaxe und Therapie von Multipler Sklerose, Morbus Crohn, Colitis ulcerosa, Rheumatoider Arthritis, Diabetes Typ I, autoimmunen Schilddrüsenerkrankungen, Morbus Wegener, Systemischem Lupus erythematosus visceralis und anderen Autoimmunerkrankungen sowie entzündlichen Erkrankungen;
- zur Prophylaxe und Therapie von allergischem Asthma bronchiale, Allergischer Rhinitis, Nahrungsmittelallergien, Atopischem Exzem, Kontaktdermatitis, Urtikaria, Angioödem und anderen allergische Erkrankungen;
- zur Prophylaxe und Therapie der Abstoßung von allogenen oder xenogenen transplantierten Organen, Geweben und Zellen, wie Nieren-, Herz-, Leber-, Pankreas-, Haut- oder Stammzelltransplantation sowie Graft versus Host-Erkrankungen;
- zur Prophylaxe und Therapie von Haut- und Schleimhauterkrankungen, wie Psoriasis, Akne sowie dermatologischen Erkrankungen mit Hyperproliferation und veränderten Differenzierungszuständen von Fibroblasten (u. a. benigner fibrosierender und sklerosierender Hauterkrankungen und maligner fibroblastärer Hyperproliferationszustände);
- zur Prophylaxe und Therapie von akuten neuronalen Erkrankungen, insbesondere Ischämie-bedingter zerebraler Schädigungen nach einem ischämischen oder hämorrhagischen Schlaganfall, Schädel/Hirn-Trauma, Herzstillstand, Herzinfarkt oder als Folge von herzchirurgischen Eingriffen;
- zur Prophylaxe und Therapie von chronischen neuronalen Erkrankungen, insbesondere Morbus Alzheimer, der Pick'schen Erkrankung, der Progressiven Supranukleären Palsy, der kortikalen Degeneration, der frontotemporalen Demenz, von Morbus Parkinson, von Morbus Huntington, von durch Prionen bedingten Erkrankungen und Amyotropher Lateralsklerose;
- zur Prophylaxe und Therapie von Atherosklerose, arterieller Entzündung, Reperfusionssyndrom und Stent-Restenose z.B. nach perkutaner transluminaler Angioplastie, auch in Form Medikament-beschichteter Stents;
- zur Prophylaxe und Therapie von Entzündungsreaktionen an oder durch in den Organismus implantierter medizin-technischer Gegenstände (medical devices), vorzugsweise in Form einer Beschichtung oder Benetzung der Gegenstände oder einer stofflichen Beimengung mindestens einer der Verbindungen oder Zusammensetzungen zum Material der Gegenstände oder in Form einer zeitlich abgestuften oder parallelen lokalen oder systemischen Gabe;
- zur Prophylaxe und Therapie von Chronisch Obstruktiven Lungenerkrankungen (COPD );
- zur Prophylaxe und Therapie von Prostatakarzinom und anderen Tumoren sowie Metastasierungen;
- zur Prophylaxe und Therapie von Schwerem Akuten Respiratorischen Syndrom (SARS); sowie
- zur Prophylaxe und Therapie von Sepsis und Sepsis-ähnlichen Zuständen.

In einem weiteren Aspekt betrifft die vorliegende Erfindung die Verwendung von mindestens einer Verbindung der allgemeinen Formel C1 bis C16 oder die Verwendung einer pharmazeutischen oder kosmetischen Zusammensetzung, die mindestens eine Verbindung der allgemeinen Formel C 1 bis C16 umfassen, zur Herstellung eines Arzneimittels zur Hemmung der Aktivität der Alanyl-Aminopeptidasen und der Dipeptidylpeptidase oder analoger Enzyme, insbesondere zur topischen Beeinflussung der Aktivität der Alanyl-Aminopeptidasen und der Dipeptidylpeptidase oder analoger Enzyme.

Darüber hinaus betrifft die vorliegende Erfindung die Verwendung von mindestens einer Verbindung der allgemeinen Formel C1 bis C16 oder die Verwendung einer pharmazeutischen oder kosmetischen Zusammensetzung, die mindestens eine Verbindung der allgemeinen Formel C 1 bis C16 umfassen, zur Herstellung eines Arzneimittels
- zur Prophylaxe und Therapie von Multipler Sklerose, Morbus Crohn, Colitis ulcerosa, Rheumatoider Arthritis, Diabetes Typ I, autoimmunen Schilddrüsenerkrankungen, Morbus Wegener, Systemischem Lupus erythematosus visceralis und anderen Autoimmunerkrankungen sowie entzündlichen Erkrankungen;
- zur Prophylaxe und Therapie von allergischem Asthma bronchiale, Allergischer Rhinitis, Nahrungsmittelallergien, Atopischem Exzem, Kontaktdermatitis, Urtikaria, Angioödem und anderen allergische Erkrankungen;
- zur Prophylaxe und Therapie der Abstoßung von allogenen oder xenogenen transplantierten Organen, Geweben und Zellen, wie Nieren-, Herz-, Leber-, Pankreas-, Haut- oder Stammzelltransplantation sowie Graft versus Host-Erkrankungen;
- zur Prophylaxe und Therapie von Haut- und Schleimhauterkrankungen, wie Psoriasis, Akne sowie dermatologischen Erkrankungen mit Hyperproliferation und veränderten Differenzierungszuständen von Fibroblasten (u. a. benigner fibrosierender und sklerosierender Hauterkrankungen und maligner fibroblastärer Hyperproliferationszustände);
- zur Prophylaxe und Therapie von akuten neuronalen Erkrankungen, insbesondere Ischämie-bedingter zerebraler Schädigungen nach einem ischämischen oder hämorrhagischen Schlaganfall, Schädel/Hirn-Trauma, Herzstillstand, Herzinfarkt oder als Folge von herzchirurgischen Eingriffen;
- zur Prophylaxe und Therapie von chronischen neuronalen Erkrankungen, insbesondere Morbus Alzheimer, der Pick'schen Erkrankung, der Progressiven Supranukleären Palsy, der kortikalen Degeneration, der frontotemporalen Demenz, von Morbus Parkinson, von Morbus Huntington, von durch Prionen bedingten Erkrankungen und Amyotropher Lateralsklerose;
- zur Prophylaxe und Therapie von Atherosklerose, arterieller Entzündung, Reperfusionssyndrom und Stent-Restenose z.B. nach perkutaner transluminaler Angioplastie, auch in Form Medikament-beschichteter Stents;
- zur Prophylaxe und Therapie von Entzündungsreaktionen an oder durch in den Organismus implantierter medizin-technischer Gegenstände (medical devices), vorzugsweise in Form einer Beschichtung oder Benetzung der Gegenstände oder einer stofflichen Beimengung mindestens einer der Verbindungen oder Zusammensetzungen zum Material der Gegenstände oder in Form einer zeitlich abgestuften oder parallelen lokalen oder systemischen Gabe;
- zur Prophylaxe und Therapie von Chronisch Obstruktiven Lungenerkrankungen (COPD);
- zur Prophylaxe und Therapie von Prostatakarzinom und anderen Tumoren sowie Metastasierungen;
- zur Prophylaxe und Therapie von Schwerem Akuten Respiratorischen Syndrom (SARS); sowie
- zur Prophylaxe und Therapie von Sepsis und Sepsis-ähnlichen Zuständen.

## Patentansprüche

1. Verbindungen der allgemeinen Formel C4 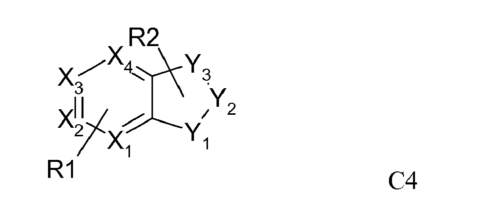 worin
• X1, X2, X3 und X4 gleich oder verschieden sein können und CH oder CR3-Einheiten darstellen;
• Y1, Y2 und Y3 gleich oder verschieden sein können substituierte oder nicht-substituierte Kohlenstoff- oder Heteroatomeinheiten mit den Ringatomen N, O, P oder S darstellen;
• R1 und R2 das Substitutionsmuster des jeweiligen Teilringes symbolisieren, wobei R1 für einen bis vier und R2 für einen bis sechs gleiche oder verschiedene Substituenten stehen, und - wie auch R3 - gewählt sind aus der Gruppe, die besteht aus Wasserstoff, unsubstituiertem oder substituiertem, geradkettigem oder verzweigtem C₁- bis C₁₂-Alkyl, C₂- bis C₁₂-Alkenyl und C₂- bis C₁₂-Alkinyl, Hydroxy, Thiol, C₁- bis C₁₂-Alkoxy, C₁- bis C₁₂-Alkylthio, unsubstituiertem oder substituiertem, unkondensiertem oder kondensiertem, gegebenenfalls ein oder mehrere Heteroatome aus der Gruppe N, O, P und S enthaltendem Aryl und Cycloalkyl, unsubstituiertem oder substituiertem Amino, unsubstituiertem oder substituiertem Carbonyl, unsubstituiertem oder substituiertem Thiocarbonyl und unsubstituiertem oder substituiertem Imino; und
• die heteroaromatischen oder heterocyclischen Reste über ein C-Atom oder ein Heteroatom mit der Grundstruktur der allgemeinen Formel C4 verbunden sind;
• und Tautomere, Stereoisomere der Verbindungen der allgemeinen Formel C4 und pharmazeutisch akzeptable Salze, Salzderivate, Tautomere und Stereoisomere davon, für die Verwendung in der Medizin.

2. Verbindungen der allgemeinen Formel C4 nach Anspruch 1 für die Verwendung in der Medizin, nämlich Verbindungen, die beispielsweise, aber nicht ausschliesslich gewählt sind aus der folgenden Gruppe C4 nach Table 4, und Tautomere, Stereoisomere der Verbindungen und pharmazeutisch akzeptable Salze, Salzderivate, Tautomere und Stereoisomere davon:
**Table 4:**
| **Compound ID.** | **Structure** |
|---|---|
| C4.002 | 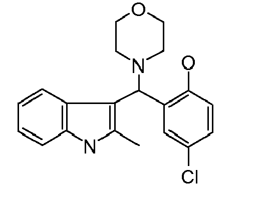 |
| C4.005 | 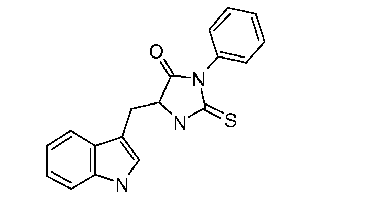 |
| C4.006 | 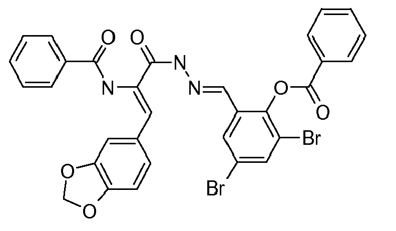 |
| C4.007 | 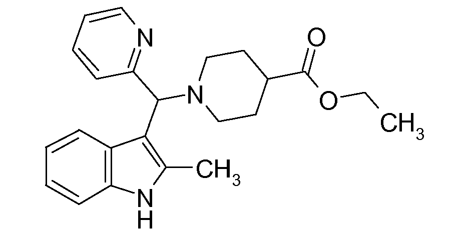 |
| C4.008 | 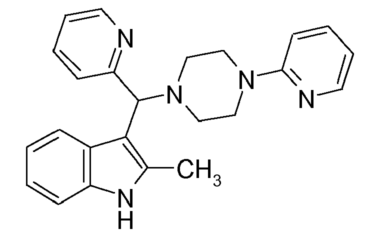 |
| C4.009 | 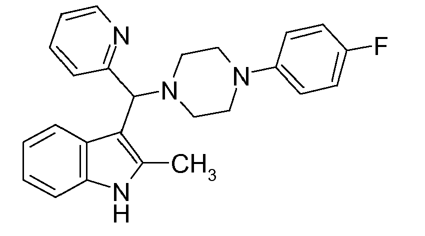 |
| C4.010 | 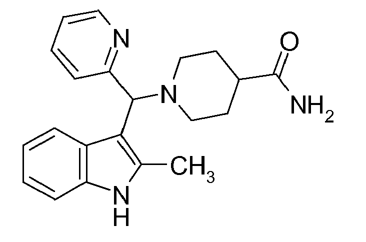 |
| C4.011 | 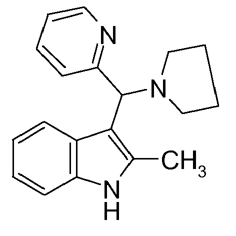 |
| C4.012 | 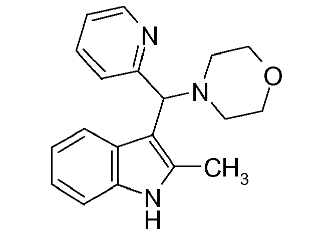 |
| C4.013 | 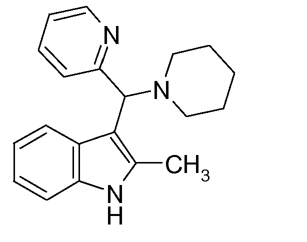 |
| C4.014 | 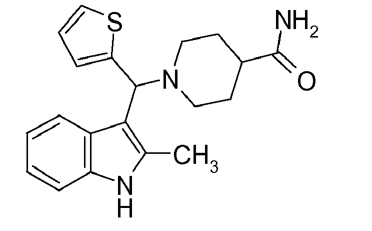 |
| C4.015 | 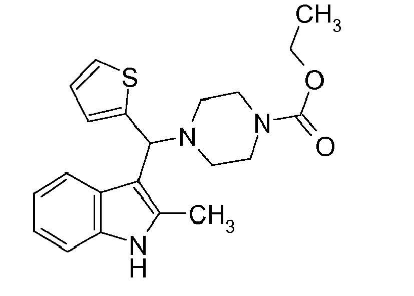 |
| C4.016 | 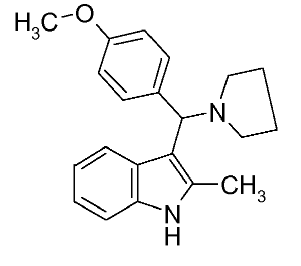 |
| C4.017 | 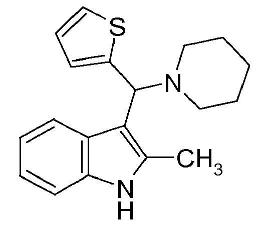 |
| C4.018 | 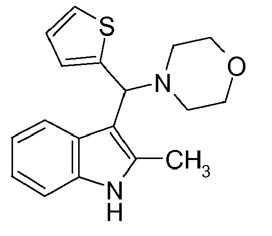 |
| C4.019 | 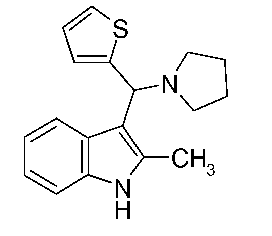 |

3. Pharmazeutische Zusammensetzung, umfassend mindestens eine Verbindung nach Anspruch 1 oder Anspruch 2, gegebenenfalls in Kombination mit an sich üblichen Trägern und/oder Adjuvantien.

4. Kosmetische Zusammensetzung, umfassend mindestens eine Verbindung nach Anspruch 1 oder Anspruch 2, gegebenenfalls in Kombination mit an sich üblichen Trägern und/oder Adjuvantien.

5. Verwendung von mindestens einer Verbindung oder pharmazeutischen oder kosmetischen Zusammensetzung nach einem der vorstehenden Ansprüche 1 bis 4 zur Hemmung der Aktivität der Alanyl-Aminopeptidasen und der Dipeptidylpeptidase oder analoger Enzyme.

6. Verwendung von mindestens einer Verbindung oder pharmazeutischen oder kosmetischen Zusammensetzung nach einem der vorstehenden Ansprüche 1 bis 4 zur topischen Beeinflussung der Aktivität der Alanyl-Aminopeptidasen und der Dipeptidylpeptidase oder analoger Enzyme.

7. Verwendung von mindestens einer Verbindung oder pharmazeutischen oder kosmetischen Zusammensetzung nach einem der vorstehenden Ansprüche 1 bis 4 zur Herstellung eines Arzneimittels zur Hemmung der Aktivität der Alanyl-Aminopeptidasen und der Dipeptidylpeptidase oder analoger Enzyme.

8. Verwendung von mindestens einer Verbindung oder pharmazeutischen oder kosmetischen Zusammensetzung nach einem der vorstehenden Ansprüche 1 bis 4 zur Herstellung eines Arzneimittels zur topischen Beeinflussung der Aktivität der Alanyl-Aminopeptidasen und der Dipeptidylpeptidase oder analoger Enzyme.

9. Verwendung von mindestens einer Verbindung oder pharmazeutischen oder kosmetischen Zusammensetzung nach einem der Ansprüche 1 bis 4 zur Herstellung eines Arzneimittels
- zur Prophylaxe und Therapie von Multipler Sklerose, Morbus Crohn, Colitis ulcerosa, Rheumatoider Arthritis, Diabetes Typ I, autoimmunen Schilddrüsenerkrankungen, Morbus Wegener, Systemischem Lupus erythematosus visceralis und anderen Autoimmunerkrankungen sowie entzündlichen Erkrankungen;
- zur Prophylaxe und Therapie von allergischem Asthma bronchiale, Allergischer Rhinitis, Nahrungsmittelallergien, Atopischem Exzem, Kontaktdermatitis, Urtikaria, Angioödem und anderen allergische Erkrankungen;
- zur Prophylaxe und Therapie der Abstoßung von allogenen oder xenogenen transplantierten Organen, Geweben und Zellen, wie Nieren-, Herz-, Leber-, Pankreas-, Haut- oder Stammzelltransplantation sowie Graft versus Host-Erkrankungen;
- zur Prophylaxe und Therapie von Haut- und Schleimhauterkrankungen, wie Psoriasis, Akne sowie dermatologischen Erkrankungen mit Hyperproliferation und veränderten Differenzierungszuständen von Fibroblasten (u. a. benigner fibrosierender und sklerosierender Hauterkrankungen und maligner fibroblastärer Hyperproliferationszustände);
- zur Prophylaxe und Therapie von akuten neuronalen Erkrankungen, insbesondere Ischämie-bedingter zerebraler Schädigungen nach einem ischämischen oder hämorrhagischen Schlaganfall, Schädel/Hirn-Trauma, Herzstillstand, Herzinfarkt oder als Folge von herzchirurgischen Eingriffen;
- zur Prophylaxe und Therapie von chronischen neuronalen Erkrankungen, insbesondere Morbus Alzheimer, der Pick'schen Erkrankung, der Progressiven Supranukleären Palsy, der kortikalen Degeneration, der frontotemporalen Demenz, von Morbus Parkinson, von Morbus Huntington, von durch Prionen bedingten Erkrankungen und Amyotropher Lateralsklerose;
- zur Prophylaxe und Therapie von Atherosklerose, arterieller Entzündung, Reperfusionssyndrom und Stent-Restenose z.B. nach perkutaner transluminaler Angioplastie, auch in Form Medikament-beschichteter Stents;
- zur Prophylaxe und Therapie von Entzündungsreaktionen an oder durch in den Organismus implantierter medizin-technischer Gegenstände (medical devices), bevorzugt in Form einer Beschichtung oder Benetzung der Gegenstände oder einer stofflichen Beimengung mindestens einer der Verbindungen oder Zusammensetzungen zum Material der Gegenstände oder in Form einer zeitlich abgestuften oder parallelen lokalen oder systemischen Gabe;
- zur Prophylaxe und Therapie von Chronisch Obstruktiven Lungenerkrankungen (COPD );
- zur Prophylaxe und Therapie von Prostatakarzinom und anderen Tumoren sowie Metastasierungen;
- zur Prophylaxe und Therapie von Schwerem Akuten Respiratorischen Syndrom (SARS); sowie
- zur Prophylaxe und Therapie von Sepsis und Sepsis-ähnlichen Zuständen.
